# EUROPEAN PATENT APPLICATION

(11) **EP 4 691 349 A1**
(43) Date of publication of application: **11.02.2026**
(21) Application number: 24848239.0
(22) Date of filing: 29.07.2024
(51) Int. Cl.: A61B 5/00

(54) **DETECTION METHOD AND WEARABLE DEVICE**

(30) Priority: 31.07.2023 CN 202310957082; 14.11.2023 CN 202311523217
(71) Applicant: Huawei Technologies Co., Ltd., Shenzhen, Guangdong 518129 (CN)
(72) Inventor: JIA, Zheng, Shenzhen, Guangdong 518129 (CN); WU, Zhouzhen, Shenzhen, Guangdong 518129 (CN); ZHU, Yu, Shenzhen, Guangdong 518129 (CN); CHEN, Qing, Shenzhen, Guangdong 518129 (CN); CHEN, Baoyang, Shenzhen, Guangdong 518129 (CN); CHEN, Qin, Shenzhen, Guangdong 518129 (CN); LI, Xu, Shenzhen, Guangdong 518129 (CN)
(74) Representative: Pfenning, Meinig & Partner mbB
(86) International application number: PCT/CN2024/108278
(87) International publication number: WO 2025/026284

(57) **Abstract**

Embodiments of this application disclose a detection method and a wearable device. The wearable device may collect physiological data (which may also be referred to as health data, including blood pressure, a heart rate, an electrocardiogram, and blood oxygen) and exercise data (including a pace, a step count, and a metabolic equivalent of task) of a user in a plurality of periods such as pre-exercise, in-exercise, and post-exercise. An exercise and health result of the user may be obtained based on the physiological data and the exercise data, so that the user can learn of a cardiovascular health status linked with everyday exercises at any time, adjust an exercise intensity, engage in safe and scientific physical training, thereby improving cardiovascular wellness.

## Description

This application claims priorities to Chinese Patent Application No. 202310957082.8, filed with the China National Intellectual Property Administration on July 31, 2023 and entitled "METHOD AND APPARATUS FOR ASSESSING CARDIOVASCULAR RISK", and to Chinese Patent Application No. 202311523217.6, filed with the China National Intellectual Property Administration on November 14, 2023 and entitled "DETECTION METHOD AND WEARABLE DEVICE", both of which are incorporated herein by reference in their entireties.

### TECHNICAL FIELD

This application relates to the field of terminal technologies, and in particular, to a detection method and a wearable device.

### BACKGROUND

With the progress of society, a physical health status like a cardiovascular health status gradually becomes one of topics of concern in people's daily life. Physical activities may affect people's physical health status like the cardiovascular health status. Moderate physical activities (for example, exercise) may reduce an incidence and an all-cause mortality rate of a cardiovascular disease. In contrast, physical activities beyond moderate intensity may increase a risk of a malignant cardiovascular event (like sudden cardiac death and myocardial infarction). Therefore, it is particularly important to detect the cardiovascular health status during physical activities.

Currently, cardiovascular health status detection related to exercise is usually performed through cardiopulmonary exercise testing (cardiopulmonary exercise testing, CPET). The CPET is an examination method that measures changes of a plurality of indicators such as gaseous metabolism, a heart rate, blood pressure, blood oxygen saturation, and an electrocardiogram in a process in which a human body changes from a resting state to a maximum force state and then returns to the resting state under gradually increasing exercise load, records corresponding symptoms of a participant during the exercise, and objectively reflects physiological and pathological changes and function damage at different load levels, to comprehensively evaluate overall functions and reserve capabilities of organ systems such as a cardiopulmonary system. However, the application of CPET is subject to stringent conditions with numerous limitations, making it unsuitable for everyday use. Therefore, in a physical activity process of daily life, how to detect a cardiovascular health status of a user is an urgent problem to be resolved.

### SUMMARY

This application provides a detection method and a wearable device. The wearable device may collect physiological data (which may also be referred to as health data, including blood pressure, a heart rate, an electrocardiogram, and blood oxygen) and exercise data (including a pace, a step count, and a metabolic equivalent of task) of a user in a plurality of periods such as pre-exercise, in-exercise, and post-exercise. An exercise and health result of the user may be obtained based on the physiological data and the exercise data, so that the user can learn of a cardiovascular health status linked with everyday exercises at any time, adjust an exercise intensity, engage in safe and scientific physical training, thereby improving cardiovascular wellness.

According to a first aspect, this application provides a detection method. The method includes: A first electronic device collects pre-exercise physiological data before a user starts a first exercise; the first electronic device collects in-exercise physiological data in a process in which the user performs the first exercise; the first electronic device collects post-exercise physiological data after the first exercise of the user ends; and the first electronic device obtains an exercise and health result of the user based on the pre-exercise physiological data, the in-exercise physiological data, and the post-exercise physiological data, and outputs the exercise and health result.

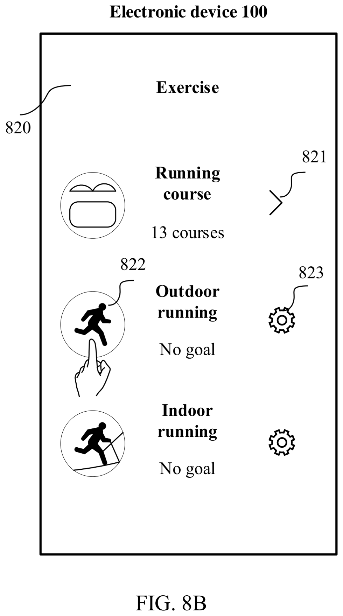

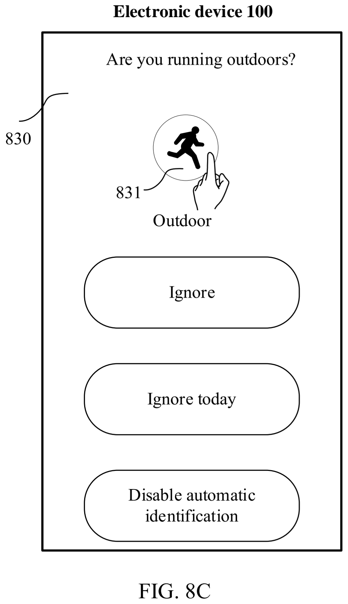

The first electronic device may be an electronic device 100 (that is, a wearable device), and the first exercise may be an exercise mode shown in FIG. 8B or FIG. 8C.

According to the method provided in the first aspect, the exercise and health result of the user can be detected, so that exercise safety monitoring can be implemented for people with a high blood pressure risk, and a suggestion or warning can be provided in time during and after the exercise of the user. In addition, the user can learn of a potential risk in time and take an active intervention measure, to avoid occurrence of a cardiovascular risk event caused by excessive exercise intensity. In addition, the user can learn of an exercise capability of a cardiopulmonary system of the user, to help the user moderately increase exercise intensity and ensure exercise effectiveness.

With reference to the first aspect, in some embodiments, the method further includes: The first electronic device displays a first report used to describe the exercise and health result, and sends the exercise and health result to a second electronic device, for the second electronic device to display a second report used to describe the exercise and health result, where content of the first report is less than content of the second report.

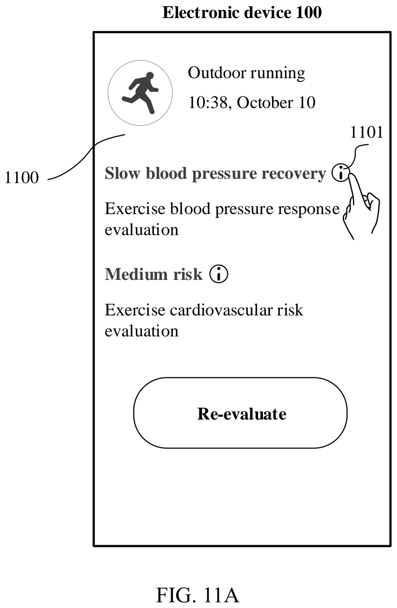

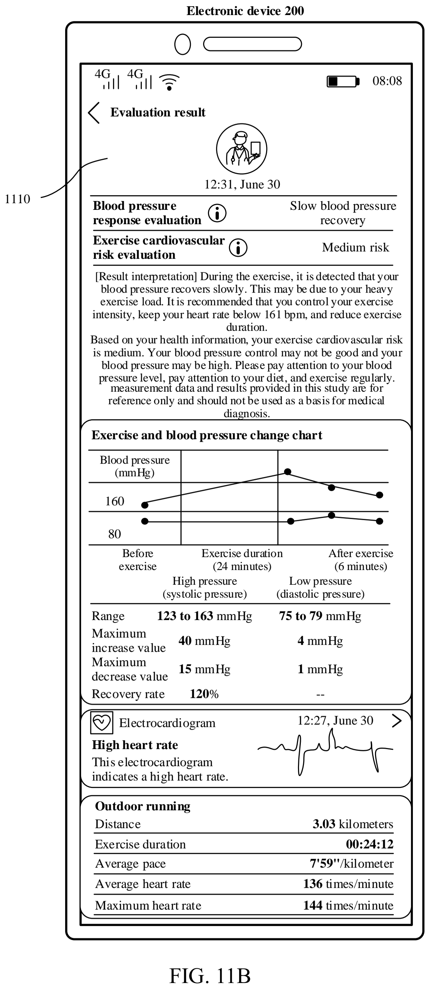

The second electronic device may be an electronic device 200 (that is, an intelligent terminal device, for example, a mobile phone), the first report may be a report displayed on a user interface 1100 shown in FIG. 11A, and the second report may be a report displayed on a user interface 1110 shown in FIG. 11B.

In this way, the first electronic device can display a brief report of the exercise and health result of the user, and may further send the exercise and health result to the second electronic device, so that the second electronic device can display a detailed report of the exercise and health result of the user. The user can view the exercise and health result on both the first electronic device and the second electronic device, enhancing user experience.

With reference to the first aspect, in some embodiments, the method further includes: Before the user starts the first exercise, the first electronic device collects the pre-exercise physiological data, and transmits the pre-exercise physiological data to the second electronic device; in the process in which the user performs the first exercise, the first electronic device collects the in-exercise physiological data, and transmits the in-exercise physiological data to the second electronic device; after the first exercise of the user ends, the first electronic device collects the post-exercise physiological data, and transmits the post-exercise physiological data to the second electronic device; and the second electronic device obtains the exercise and health result of the user based on the pre-exercise physiological data, the in-exercise physiological data, and the post-exercise physiological data, and outputs the exercise and health result.

In this way, the first electronic device may establish a communication connection to the second electronic device, and synchronously send the collected pre-exercise physiological data, the collected in-exercise physiological data, and the collected post-exercise physiological data to the second electronic device through the communication connection. The second electronic device may obtain the exercise and health result of the user based on the physiological data, and output the exercise and health result.

With reference to the first aspect, in some embodiments, the first electronic device includes a wearable device, and a display of the second electronic device is larger than a display of the first electronic device.

In this way, the second electronic device can better display the detailed report of the exercise and health result of the user.

With reference to the first aspect, in some embodiments, the method further includes: The first electronic device displays a first prompt message in the process in which the user performs the first exercise, where the first prompt message indicates a health abnormality in the process in which the user performs the first exercise, and the first prompt message is generated based on the in-exercise physiological data, or is generated based on the pre-exercise physiological data and the in-exercise physiological data.

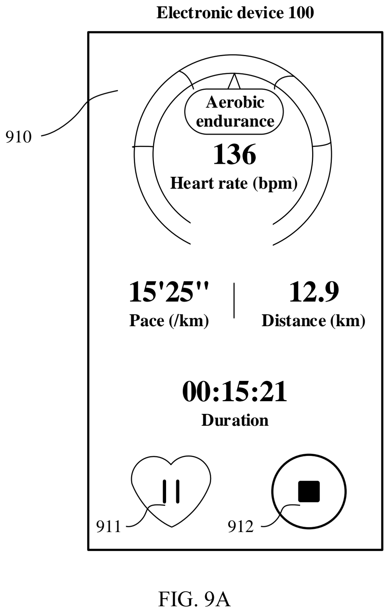

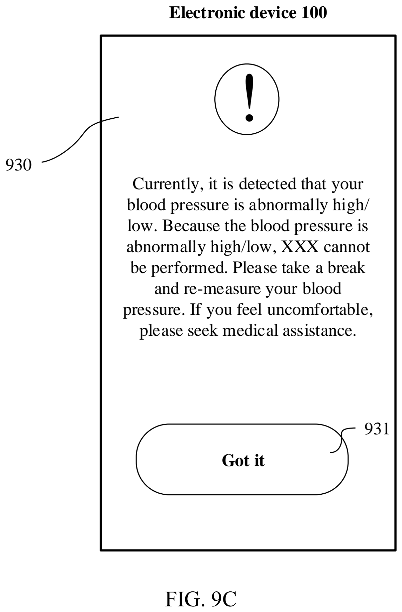

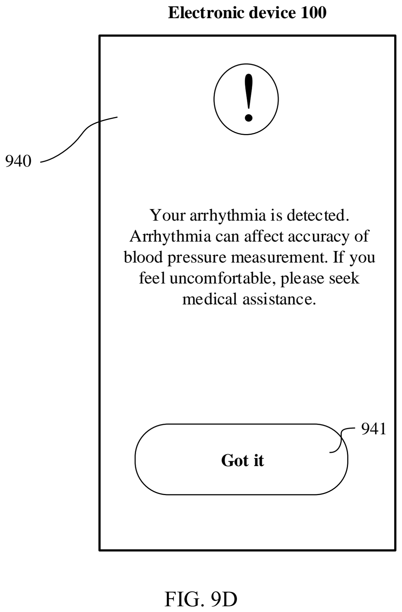

The first prompt message may be prompt information on a user interface 930 shown in FIG. 9C or a user interface 940 shown in FIG. 9D.

In this way, exercise safety monitoring can be implemented for people with a high blood pressure risk, and a suggestion or warning can be provided in time during the exercise of the user.

With reference to the first aspect, in some embodiments, the health abnormality includes one or more of the following: an abnormally high blood pressure, an abnormally low blood pressure, a retarded reaction, arrhythmia, an abnormal ST segment change, and an abnormal blood oxygen decrease.

With reference to the first aspect, in some embodiments, the exercise and health result includes a blood pressure evaluation result, and the blood pressure evaluation result is generated based on the pre-exercise physiological data, the in-exercise physiological data, and the post-exercise physiological data, or is generated based on the pre-exercise physiological data and the post-exercise physiological data.

With reference to the first aspect, in some embodiments, the exercise and health result includes cardiovascular risk prediction, and the cardiovascular risk prediction is generated based on two or three of the pre-exercise physiological data, the in-exercise physiological data, and the post-exercise physiological data, and one or more of an exercise mode, a pre-exercise questionnaire, and a post-exercise questionnaire.

With reference to the first aspect, in some embodiments, the process in which the user performs the first exercise includes an exercise interval of the user performing the first exercise.

With reference to the first aspect, in some embodiments, before collecting the pre-exercise physiological data, the method further includes: The first electronic device obtains an exercise mode of the user, and selects, based on the exercise mode, an algorithm model used to analyze the exercise and health result, where the exercise mode includes the first exercise. That the first electronic device obtains the exercise and health result of the user based on the pre-exercise physiological data, the in-exercise physiological data, and the post-exercise physiological data specifically includes: The first electronic device analyzes the pre-exercise physiological data, the in-exercise physiological data, and the post-exercise physiological data by using the algorithm model, to obtain the exercise and health result of the user.

In this way, the first electronic device can analyze the pre-exercise physiological data, the in-exercise physiological data, and the post-exercise physiological data by using the algorithm model corresponding to the first exercise, to obtain the exercise and health result of the user, so that the obtained exercise and health result of the user is more accurate and better meets an actual situation of the user.

With reference to the first aspect, in some embodiments, that the first electronic device obtains the exercise mode of the user specifically includes: The first electronic device obtains the exercise mode based on an input of the user; or the first electronic device collects exercise data of the user when the user starts the first exercise, and then determines the exercise mode based on the exercise data.

In this way, the first electronic device can obtain the exercise mode of the user based on the input of the user or by analyzing the collected exercise data. The exercise mode of the user is determined based on the exercise data collected by the first electronic device, so that the user can reduce an input operation, the user does not need to manually select the exercise mode, and user experience is improved.

With reference to the first aspect, in some embodiments, the method further includes: The first electronic device displays a first user interface if it is detected that the in-exercise physiological data deviates from normal physiological data of the user, where the first user interface includes a second prompt, the second prompt indicates a risk that exists when the user performs the first exercise, and the normal physiological data is a reference value that is of physiological data when the user performs the first exercise and that is determined based on the pre-exercise physiological data.

The first user interface may be the user interface 930 shown in FIG. 9C or the user interface 940 shown in FIG. 9D.

In this way, a larger deviation of the in-exercise physiological data from the normal physiological data in the exercise mode indicates a higher risk level of performing the first exercise by the user. In this way, exercise safety monitoring can further be implemented for people with a high blood pressure risk, and a suggestion or warning can be provided in time during the exercise of the user.

With reference to the first aspect, in some embodiments, the exercise and health result further includes first evaluation, and the first evaluation indicates an exercise suggestion for the user to perform the first exercise.

With reference to the first aspect, in some embodiments, the method further includes: The first electronic device obtains, based on the exercise data in the first exercise process and the in-exercise physiological data, exercise and health results obtained when the user is in different exercise states in the process of performing the first exercise, where the first evaluation specifically indicates an exercise status that is suitable for or not suitable for the user in the different exercise states.

In this way, the first electronic device may obtain, based on the in-exercise physiological data and the exercise data, the exercise and health results of the user in different exercise states during the exercise, to obtain an in-exercise exercise and health suggestion for the user. Further, the user can learn of an exercise capability of a cardiopulmonary system of the user, to help the user moderately increase exercise intensity and ensure exercise effectiveness.

With reference to the first aspect, in some embodiments, the method further includes: The first electronic device obtains, based on historical physiological data and historical exercise data, an exercise suggestion before the first exercise starts, where the historical physiological data includes physiological data collected before a previous exercise of the first exercise starts, physiological data collected in a process of the previous exercise, and physiological data collected after the previous exercise ends, the historical exercise data includes exercise data of the previous exercise, the exercise suggestion before the first exercise starts includes second evaluation, and the second evaluation indicates whether the user is suitable for or not suitable for the first exercise.

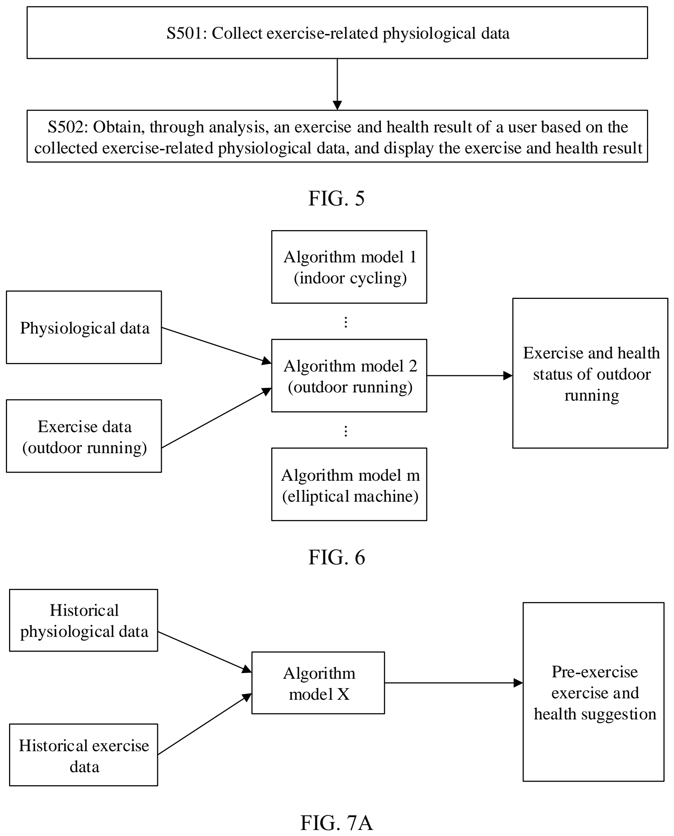

The second evaluation may be a pre-exercise exercise and health suggestion shown in FIG. 7A.

In this way, the first electronic device can obtain, based on the historical physiological data and the historical exercise data, a pre-exercise exercise and health suggestion, so that the first electronic device can provide, before the exercise, a suggestion or a warning for an exercise mode selected by the user and physiological data that the user needs to pay attention to in a current exercise process, to avoid occurrence of a cardiovascular risk event caused because the user is not suitable for the selected exercise mode.

With reference to the first aspect, in some embodiments, the in-exercise physiological data includes one or more of the following: a heart rate, blood pressure, an electrocardiogram, and blood oxygen. That the first electronic device collects the in-exercise physiological data in the process in which the user performs the first exercise specifically includes: The first electronic device displays a second user interface in the process in which the user performs the first exercise, where the second user interface includes exercise data and a heart rate that are in the process in which the user performs the first exercise, and the second user interface further includes an exercise pause control; and the first electronic device detects an operation of tapping the exercise pause control by the user, suspends collecting the exercise data, and displays a third user interface, where the third user interface includes one or more of the following options: a blood pressure measurement option, an electrocardiogram collection option, and a blood oxygen measurement option.

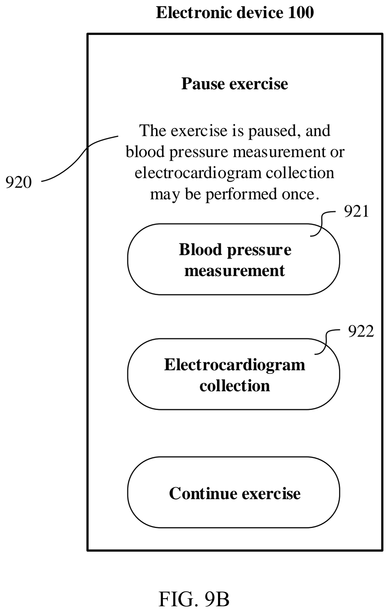

The second user interface may be a user interface 910 shown in FIG. 9A, and the third user interface may be a user interface 920 shown in FIG. 9B.

In this way, the first electronic device can trigger, based on the input of the user, collection of the in-exercise physiological data during the exercise of the user.

With reference to the first aspect, in some embodiments, "after the first exercise of the user ends" is specifically "before first duration after the first exercise of the user ends".

The first duration may be duration greater than or equal to duration of an exercise recovery period, and the duration of the exercise recovery period is duration in which the post-exercise physiological data gradually recovers to the resting level.

With reference to the first aspect, in some embodiments, the post-exercise physiological data includes one or more of the following: a heart rate, blood pressure, an electrocardiogram, and blood oxygen. That the first electronic device collects the post-exercise physiological data after the first exercise of the user ends specifically includes: The second user interface further includes an exercise end control; the first electronic device detects an operation of tapping the exercise end control by the user, ends collecting the exercise data, and displays a fourth user interface, where the fourth user interface includes a countdown control, duration of the countdown control is set to second duration, and the second duration is less than or equal to the first duration; and the first electronic device displays a fifth user interface when timing of the countdown control ends, where the fifth user interface includes one or more of the following options: a blood pressure measurement option, a heart rate measurement option, an electrocardiogram collection option, and a blood oxygen measurement option.

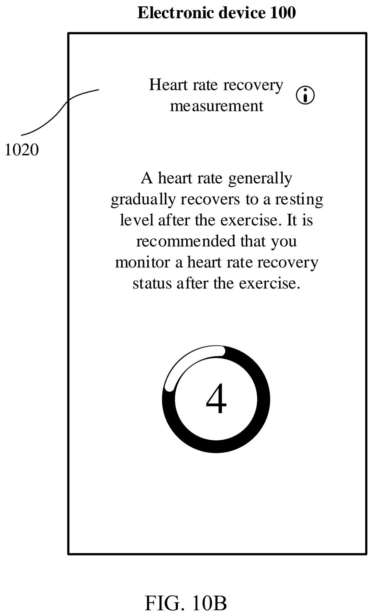

The second duration may be duration less than or equal to the first duration, and the fourth user interface may be a user interface 1020 shown in FIG. 10B.

In this way, the first electronic device can trigger, based on the input of the user, collection of the post-exercise physiological data after the exercise of the user.

With reference to the first aspect, in some embodiments, outputting the exercise and health result specifically includes: displaying one or more of the following risk factor items: a risk factor item evaluated based on the in-exercise physiological data and a risk factor item evaluated based on the post-exercise physiological data; and displaying a comprehensive risk level of performing the first exercise by the user, where the comprehensive risk level is determined based on a risk level of the risk factor item evaluated based on the in-exercise physiological data and a risk level of the risk factor item evaluated based on the post-exercise physiological data.

In this way, the exercise and health result of the user can be presented in terms of a risk level, a risk factor, and the like.

According to a second aspect, this application provides an exercise and health detection method, applied to a first communication system. The first communication system includes a first electronic device and a second electronic device. The method includes: Before a user starts a first exercise, the first electronic device collects first physiological data, and transmits the first physiological data to the second electronic device; the first electronic device collects second physiological data in a process in which the user performs the first exercise, and transmits the second physiological data to the second electronic device; the first electronic device collects third physiological data after the first exercise of the user ends, and transmits the third physiological data to the second electronic device; and the second electronic device obtains an exercise and health status of the user based on the first physiological data, the second physiological data, and the third physiological data through analysis, and displays the exercise and health status, where the exercise and health status includes first evaluation and second evaluation, the first evaluation indicates an exercise suggestion for the user to perform the first exercise, and the second evaluation indicates a physical health recovery status of the user after the first exercise ends.

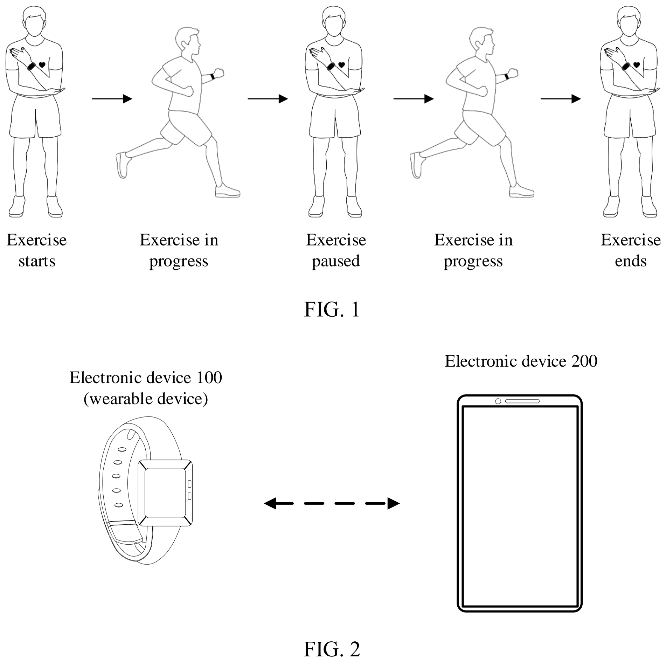

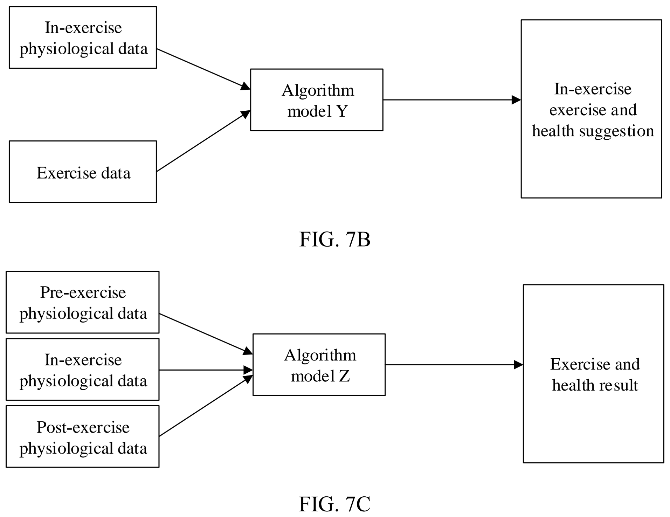

The first communication system may be a communication system shown in FIG. 2, the first electronic device may be an electronic device 100 (that is, a wearable device), the second electronic device may be an electronic device 200 (that is, an intelligent terminal device, for example, a mobile phone), the first exercise may be an exercise mode shown in FIG. 8B or FIG. 8C, the first physiological data may be pre-exercise physiological data, the second physiological data may be in-exercise physiological data, the third physiological data may be post-exercise physiological data, the first evaluation may be an in-exercise exercise and health suggestion shown in FIG. 7B, and the second evaluation may be a post-exercise exercise and health suggestion shown in FIG. 7C.

According to the method provided in the first aspect, the exercise and health status of the user can be detected, so that exercise safety monitoring can be implemented for people with a high blood pressure risk, and a suggestion or warning can be provided in time during and after the exercise of the user. In addition, the user can learn of a potential risk in time and take an active intervention measure, to avoid occurrence of a cardiovascular risk event caused by excessive exercise intensity. In addition, the user can learn of an exercise capability of a cardiopulmonary system of the user, to help the user moderately increase exercise intensity and ensure exercise effectiveness.

With reference to the second aspect, in some embodiments, after displaying the exercise and health status, the method further includes: The second electronic device sends, to the first electronic device, the exercise and health status that is of the user and that is obtained through analysis, and the first electronic device displays the exercise and health status.

In this way, after displaying the exercise and health status of the user, the second electronic device may send, to the first electronic device, the exercise and health status obtained through analysis, so that the first electronic device can also display the exercise and health status of the user. The user can view the exercise and health status on both the first electronic device and the second electronic device, enhancing user experience.

With reference to the second aspect, in some embodiments, the first electronic device includes a wearable device, and a display of the second electronic device is larger than a display of the first electronic device.

In this way, the second electronic device can better display the exercise and health status of the user.

With reference to the second aspect, in some embodiments, the process in which the user performs the first exercise includes an exercise interval of the user performing the first exercise.

With reference to the second aspect, in some embodiments, before collecting the first physiological data, the method further includes: The first electronic device obtains an exercise mode of the user; and the second electronic device selects, based on the exercise mode, an algorithm model used to analyze the exercise and health status, where the exercise mode includes the first exercise. That the second electronic device obtains the exercise and health status of the user by analyzing the first physiological data, the second physiological data, and the third physiological data specifically includes: The second electronic device analyzes the first physiological data, the second physiological data, and the third physiological data by using the algorithm model, to obtain the exercise and health status of the user.

In this way, the second electronic device can analyze the first physiological data, the second physiological data, and the third physiological data by using the algorithm model corresponding to the first exercise, to obtain the exercise and health status of the user, so that the obtained exercise and health status of the user is more accurate and better meets an actual situation of the user.

With reference to the second aspect, in some embodiments, that the first electronic device obtains the exercise mode of the user specifically includes: The first electronic device obtains the exercise mode based on an input of the user; or the first electronic device collects exercise data of the user when the user starts the first exercise, and then obtains the exercise mode through analysis based on the exercise data.

In this way, the first electronic device can obtain the exercise mode of the user based on the input of the user or by analyzing the collected exercise data. The exercise mode of the user is obtained through analysis based on the exercise data collected by the first electronic device, so that the user can reduce an input operation, the user does not need to manually select the exercise mode, and user experience is improved.

With reference to the second aspect, in some embodiments, the first evaluation is determined based on the first physiological data and the second physiological data. If the second physiological data deviates from normal physiological data of the user, the first evaluation indicates that the user is not suitable for the first exercise; otherwise, the first evaluation indicates that the user is suitable for the first exercise. The normal physiological data is a reference value that is of physiological data when the user performs the first exercise and that is determined based on the first physiological data.

In this way, the second electronic device can determine, based on the first physiological data and the second physiological data, whether the second physiological data deviates from the normal physiological data of the user, so that an evaluation result indicating whether the user is suitable for the first exercise can be obtained.

With reference to the second aspect, in some embodiments, a degree to which the second physiological data deviates from the normal physiological data in the exercise mode indicates a risk level of performing the first exercise by the user.

In this way, a larger deviation of the second physiological data from the normal physiological data in the exercise mode indicates a higher risk level of performing the first exercise by the user. In this way, exercise safety monitoring can further be implemented for people with a high blood pressure risk, and a suggestion or warning can be provided in time during the exercise of the user.

With reference to the second aspect, in some embodiments, after the second physiological data deviates from the normal physiological data of the user, the method further includes: displaying a first user interface, where the first user interface includes prompt information, the prompt information is used to prompt the user of a risk that exists when the user performs the first exercise, and the first user interface further includes one or more of the following options: a blood pressure measurement option and an electrocardiogram collection option.

The first user interface may be a user interface 930 shown in FIG. 9C.

In this way, after detecting that the second physiological data deviates from the normal physiological data of the user during the exercise of the user, the first electronic device can perform an abnormal event triggering reminder, so that the user learns of a potential risk in time and takes an active intervention measure, to avoid occurrence of a cardiovascular risk event caused by excessively high exercise intensity.

With reference to the second aspect, in some embodiments, before transmitting the second physiological data to the second electronic device, the method further includes: The first electronic device collects the exercise data in the process in which the user performs the first exercise, and transmits the exercise data to the second electronic device.

In this way, the exercise data can be collected during the exercise of the user, so that the second electronic device can combine the exercise data and the second physiological data for analysis, to obtain an in-exercise exercise and health suggestion of the user. The second electronic device may further combine the exercise data with the first physiological data, the second physiological data, and the third physiological data for analysis, to obtain a post-exercise exercise and health suggestion and a cardiovascular risk evaluation result of the user.

With reference to the second aspect, in some embodiments, after transmitting second physiological data to the second electronic device, the method further includes: The second electronic device obtains, through analysis based on the exercise data and the second physiological data, exercise and health statuses of the user in different exercise states in the process of performing the first exercise, where the first evaluation indicates an exercise state that is suitable for or not suitable for the user in the different exercise states.

In this way, the second electronic device may obtain through analysis, based on the second physiological data and the exercise data, exercise and health statuses of the user in different exercise states during the exercise, to obtain an in-exercise exercise and health suggestion for the user. Further, the user can learn of an exercise capability of a cardiopulmonary system of the user, to help the user moderately increase exercise intensity and ensure exercise effectiveness.

With reference to the second aspect, in some embodiments, after the first electronic device obtains the exercise mode of the user, the method further includes: The second electronic device obtains through analysis, based on historical physiological data and historical exercise data, an exercise suggestion before the first exercise starts, where the historical physiological data includes physiological data collected before a previous exercise of the first exercise starts, physiological data collected in a process of the previous exercise, and physiological data collected after the previous exercise ends, the historical exercise data includes exercise data of the previous exercise, the exercise suggestion before the first exercise starts includes third evaluation, and the third evaluation indicates whether the user is suitable for or not suitable for the first exercise.

The third evaluation may be a pre-exercise exercise and health suggestion shown in FIG. 7A.

In this way, the second electronic device can obtain through analysis, based on the historical physiological data and the historical exercise data, a pre-exercise exercise and health suggestion, so that the second electronic device can provide, before the exercise, a suggestion or a warning for an exercise mode selected by the user and physiological data that the user needs to pay attention to in a current exercise process, to avoid occurrence of a cardiovascular risk event caused because the user is not suitable for the selected exercise mode.

With reference to the second aspect, in some embodiments, that the first electronic device collects the first physiological data before the user starts the first exercise specifically includes: The first electronic device triggers collection of the first physiological data based on an input of the user, or the first electronic device collects the first physiological data when detecting that the user is in a non-exercise state.

In this way, the first electronic device can trigger, based on the input of the user, collection of the first physiological data before the exercise of the user, or collection of the first physiological data when detecting that the user is in the non-exercise state, enhancing user experience.

With reference to the second aspect, in some embodiments, that the first electronic device collects the second physiological data in the process in which the user performs the first exercise specifically includes: The first electronic device triggers collection of the second physiological data based on an input of the user, or the first electronic device collects the second physiological data when detecting that the user is in an exercise state.

In this way, the first electronic device can trigger, based on the input of the user, collection of the second physiological data during the exercise of the user, or collection of the second physiological data when detecting that the user is in the exercise state, enhancing user experience.

With reference to the second aspect, in some embodiments, the second physiological data includes one or more of the following: a heart rate, blood pressure, and an electrocardiogram. That the first electronic device triggers, based on the input of the user, collection of the second physiological data specifically includes: The first electronic device displays a second user interface in the process in which the user performs the first exercise, where the second user interface includes exercise data and a heart rate that are in the process in which the user performs the first exercise, and the second user interface further includes an exercise pause control; and the first electronic device detects an operation of tapping the exercise pause control by the user, suspends collecting the exercise data, and displays a third user interface, where the third user interface includes one or more of the following options: a blood pressure measurement option and an electrocardiogram collection option.

The second user interface may be a user interface 910 shown in FIG. 9A, and the third user interface may be a user interface 920 shown in FIG. 9B.

In this way, the first electronic device can trigger, based on the input of the user, collection of the second physiological data during the exercise of the user.

With reference to the second aspect, in some embodiments, that the first electronic device collects the third physiological data after the first exercise of the user ends specifically includes: The first electronic device triggers collection of the third physiological data based on an input of the user, or the first electronic device collects the third physiological data after detecting that the exercise of the user ends.

In this way, the first electronic device can trigger, based on the input of the user, collection of the third physiological data after the exercise of the user, or collection of the third physiological data after detecting that the exercise of the user ends, enhancing user experience.

With reference to the second aspect, in some embodiments, "after the first exercise of the user ends" is specifically "before first duration after the first exercise of the user ends".

The first duration may be duration greater than or equal to duration of an exercise recovery period, and the duration of the exercise recovery period is duration in which the third physiological data gradually recovers to the resting level.

With reference to the second aspect, in some embodiments, the third physiological data includes one or more of the following: a heart rate, blood pressure, and an electrocardiogram. That the first electronic device triggers collection of the third physiological data based on the input of the user specifically includes: The second user interface further includes an exercise end control; the first electronic device detects an operation of tapping the exercise end control by the user, ends collecting the exercise data, and displays a fourth user interface, where the fourth user interface includes a countdown control, duration of the countdown control is set to second duration, and the second duration is less than or equal to the first duration; and the first electronic device displays a fifth user interface when timing of the countdown control ends, where the fifth user interface includes one or more of the following options: a blood pressure measurement option, a heart rate measurement option, and an electrocardiogram collection option.

The second duration may be duration less than or equal to the first duration, and the fourth user interface may be a user interface 1020 shown in FIG. 10B.

In this way, the first electronic device can trigger, based on the input of the user, collection of the third physiological data after the exercise of the user.

With reference to the second aspect, in some embodiments, displaying the exercise and health result specifically includes: displaying one or more of the following risk factor items: a risk factor item evaluated based on the second physiological data and a risk factor item evaluated based on the third physiological data; and displaying a comprehensive risk level of performing the first exercise by the user, where the comprehensive risk level is determined based on a risk level of the risk factor item evaluated based on the second physiological data and a risk level of the risk factor item evaluated based on the third physiological data.

In this way, the exercise and health status of the user can be presented in terms of a risk level, a risk factor, and the like.

With reference to the second aspect, in some embodiments, the first evaluation specifically includes an exercise suggestion of the user in the process of performing the first exercise, and the second evaluation specifically includes an exercise suggestion of the user after the first exercise ends.

In this way, the exercise and health status of the user at different stages can be presented.

With reference to the second aspect, in some embodiments, after the displaying the exercise and health status, the method further includes: displaying exercise and health statuses of a plurality of exercises performed by the user, where the plurality of exercises include the first exercise.

In this way, the exercise and health status of the user can be presented in terms of measurement and statistics.

With reference to the second aspect, in some embodiments, the exercise and health statuses of the plurality of exercises includes one or more of the following: quantities of occurrences of different risk levels in the plurality of exercises, a risk level of each exercise, and physiological data collected during or after each exercise.

According to a third aspect, this application provides an electronic device, where the electronic device includes a processor and a memory. The memory is coupled to the processor, the memory is configured to store computer program code, the computer program code includes computer instructions, and when the processor executes the computer instructions, the electronic device is enabled to perform the method according to any one of the implementations of the first aspect.

According to a fourth aspect, this application provides a computer-readable storage medium. The computer-readable storage medium stores a computer program or computer instructions, and the computer program or the computer instructions are executed by a processor to implement the method according to any one of the implementations of the first aspect.

According to a fifth aspect, this application provides a computer program product. When the computer program product is executed by a processor, the method according to any one of the implementations of the first aspect is implemented.

According to a sixth aspect, this application provides a chip, where the chip includes a processor and a memory. The memory is configured to store a computer program or computer instructions, and the processor is configured to execute the computer program or the computer instructions stored in the memory, to enable the chip to perform the method according to any one of the possible implementations of the first aspect.

The solutions provided in the second aspect to the sixth aspect are used to implement or cooperate to implement the method provided in the first aspect, and therefore, can achieve beneficial effects the same as or corresponding to those in the first aspect. Details are not described herein again.

### BRIEF DESCRIPTION OF DRAWINGS

FIG. 1 is a diagram of a scenario in which exercise-related physiological data of a user is collected according to an embodiment of this application;
FIG. 2 is a diagram of a communication system according to an embodiment of this application;
FIG. 3 is a diagram of a structure of an electronic device 100 according to an embodiment of this application;
FIG. 4 is a diagram of a structure of an electronic device 200 according to an embodiment of this application;
FIG. 5 is a schematic flowchart of a detection method according to an embodiment of this application;
FIG. 6 is a diagram of a method for selecting an algorithm model corresponding to an exercise mode to analyze an exercise and health result according to an embodiment of this application;
FIG. 7A is a diagram of a method for providing an exercise and health suggestion based on an algorithm model corresponding to pre-exercise according to an embodiment of this application;
FIG. 7B is a diagram of a method for providing an exercise and health suggestion based on an algorithm model corresponding to in-exercise according to an embodiment of this application;
FIG. 7C is a diagram of a method for obtaining an exercise and health result based on an algorithm model corresponding to a whole exercise process according to an embodiment of this application;
FIG. 8A to FIG. 8K are diagrams of a group of interfaces according to an embodiment of this application;
FIG. 9A to FIG. 9D are diagrams of another group of interfaces according to an embodiment of this application;
FIG. 10A to FIG. 10J are diagrams of another group of interfaces according to an embodiment of this application;
FIG. 11A and FIG. 11B are diagrams of another group of interfaces according to an embodiment of this application;
FIG. 12A to FIG. 12C are diagrams of another group of interfaces according to an embodiment of this application;
FIG. 13A to FIG. 13D are diagrams of another group of interfaces according to an embodiment of this application;
FIG. 14 is a diagram of a curve of a relationship between a pulse wave transmission speed and an age and a location of a user within population distribution according to an embodiment of this application; and
FIG. 15 is a diagram of a fitting curve of a relationship between blood pressure and a heart rate according to an embodiment of this application.

### DESCRIPTION OF EMBODIMENTS

Terms used in embodiments of this application are merely intended to describe specific embodiments, but are not intended to limit this application.

Physical activities may affect people's physical health status like a cardiovascular health status. Moderate physical activities (for example, exercise) may improve cardiopulmonary endurance, reduce an incidence and an all-cause mortality rate of a cardiovascular disease. Although an absolute risk is low, physical activities beyond moderate intensity may also increase a risk of a malignant cardiovascular event (like sudden cardiac death and myocardial infarction).

Currently, cardiovascular health status detection related to exercise is usually performed through cardiopulmonary exercise testing (cardiopulmonary exercise testing, CPET). The CPET is an examination method that measures changes of a plurality of indicators such as gaseous metabolism, a heart rate, blood pressure, blood oxygen saturation, and an electrocardiogram in a process in which a human body changes from a resting state to a maximum force state and then returns to the resting state under gradually increasing exercise load, records corresponding symptoms of a participant during the exercise, and objectively reflects physiological and pathological changes and function damage at different load levels, to comprehensively evaluate overall functions and reserve capabilities of organ systems such as a cardiopulmonary system. As an objective, quantitative, and non-invasive examination method, the CPET has been used in clinic for more than half a century. The CPET can clarify a pathophysiological mechanism of related symptoms in exercise intolerance and an exercise state, and has important clinical value in disease differential diagnosis, prognosis evaluation, medical intervention effect evaluation, and exercise prescription formulation.

It can be learned from the foregoing existing solution that the application of the CPET is subject to stringent conditions. The CPET can be used to detect a cardiovascular health status related to exercise only after being used in a professional organization, with a professional device, and under guidance of a professional, making it unsuitable for everyday use. In addition, this cardiovascular health status detection manner has many limitations, and can be performed only under gradually increasing exercise load.

In view of the foregoing problems, embodiments of this application provide a detection method and a wearable device.

As shown in FIG. 1, in this embodiment of this application, the wearable device may collect physiological data (which may also be referred to as health data, including blood pressure, a heart rate, an electrocardiogram, and blood oxygen) and exercise data (including a pace, a step count, and a metabolic equivalent of task) of a user in a plurality of periods such as pre-exercise, in-exercise, and post-exercise. An exercise and health result of the user may be obtained based on the physiological data and the exercise data, so that the user can learn of a cardiovascular health status linked with everyday exercises at any time, adjust an exercise intensity, engage in safe and scientific physical training, thereby improving cardiovascular wellness.

First, a communications system and a related device provided in embodiments of this application are described.

**FIG. 2** **shows an example of a communication system according to an embodiment of this application.**

As shown in FIG. 2, the communication system may include an electronic device 100 (wearable device) and an electronic device 200.

A communication connection is established between the electronic device 100 and the electronic device 200. The communication connection may be, for example, a Bluetooth (Bluetooth, BT) communication connection or a near field communication (near field communication, NFC) connection. This is not limited thereto. The communication connection may alternatively be, for example, a wireless local area network (wireless local area network, WLAN) communication connection, or a cellular mobile communication connection. If the communication connection is a WLAN communication connection or a cellular mobile communication connection, the communication connection between the electronic device 100 and the electronic device 200 may be an indirect communication connection, that is, a communication connection established by using an access point (access point, AP) or the like. The AP may include but is not limited to a base station or a router.

The electronic device 100 may be configured to measure physiological data by using a micro pump, an airbag, a barometric pressure sensor, a PPG (photoplethysmography, photoplethysmography) sensor, an ECG (Electrocardiography, electrocardiography, which may be equivalent to electrocardiogram in embodiments of this application) sensor, a blood oxygen sensor, and the like, and may be further configured to measure exercise data by using an acceleration sensor, a gyroscope sensor, a positioning sensor, a timing sensor, and the like. The physiological data may include but is not limited to blood pressure, a heart rate, an electrocardiogram, and blood oxygen. The exercise data may include but is not limited to an exercise speed, an exercise distance, exercise duration, an exercise direction, a location, an altitude, a pace, a step count, and a metabolic equivalent of task.

In some embodiments, the electronic device 100 may be further configured to: obtain an exercise and health result of the user based on the measured physiological data and the measured exercise data, and output the exercise and health result.

In some other embodiments, the electronic device 100 may establish a communication connection to the electronic device 200, and the electronic device 100 may be configured to send the measured physiological data and the measured exercise data to the electronic device 200 through the communication connection to the electronic device 200, so that the electronic device 200 obtains the exercise and health result of the user.

The electronic device 200 may be configured to: receive the physiological data and the exercise data from the electronic device 100, obtain the exercise and health result of the user based on the physiological data and the exercise data, and display the exercise and health result of the user through a display or the like. The electronic device 200 may further provide an online doctor diagnosis function, for example, send the physiological data and the exercise data of the user to a server that provides cloud diagnosis, and trigger the server to provide a cloud diagnosis service.

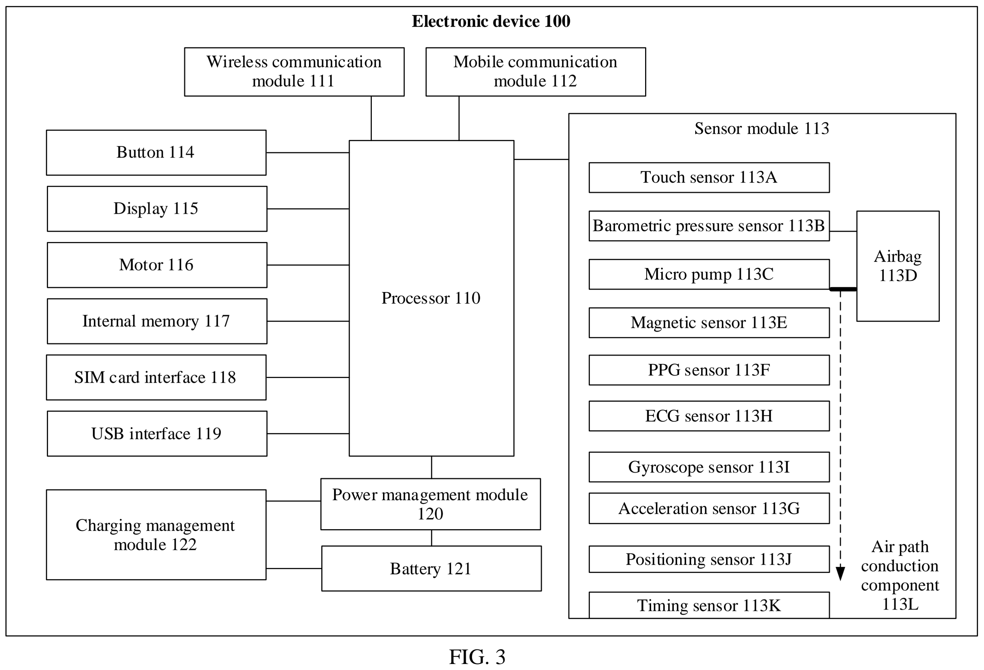

**FIG. 3** **shows an example of an electronic device 100 according to an embodiment of this application.**

The electronic device 100 may be portablely worn by a user, and is applicable to a plurality of exercise scenarios such as indoor and outdoor exercise scenarios. In addition, the electronic device 100 has a capability of monitoring vital signs such as blood pressure, a heart rate, and an electrocardiogram, and a capability of monitoring exercise indicators such as a pace, a step count, and a distance. The electronic device 100 may be a smartwatch, a smart band, or the like. A specific type of the electronic device 100 is not specially limited in this embodiment of this application. In this embodiment of this application, an example in which the electronic device 100 is a watch is used for description.

As shown in FIG. 3, the electronic device 100 may include a processor 110, a wireless communication module 111, a mobile communication module 112, a sensor module 113, a button 114, a display 115, a motor 116, an internal memory 117, a subscriber identity module (subscriber identity module, SIM) card interface 118, a universal serial bus (universal serial bus, USB) interface 119, a power management module 120, a battery 121, and a charging management module 122. The sensor module 113 may include a touch sensor 113A, a barometric pressure sensor 113B, a micro pump 113C, an airbag 113D, a magnetic sensor 113E, a PPG sensor 113F, an ECG sensor 113H, a gyroscope sensor 113I, an acceleration sensor 113G, a positioning sensor 113J, a timing sensor 113K, and an air path conduction component 113L.

It may be understood that the structure shown in this embodiment of the present invention does not constitute a specific limitation on the electronic device 100. In some other embodiments of this application, the electronic device 100 may include more or fewer components than those shown in the figure, or a combination of some components, or splits from some components, or a different component layout. The components shown in the figure may be implemented by using hardware, software, or a group of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a SIM interface, a USB interface, and/or the like.

In some embodiments, the processor 110 may alternatively be a microcontroller unit (mircrocontroller unit, MCU).

The I2C interface is a two-way synchronization serial bus, and includes one serial data line (serial data line, SDA) and one serial clock line (serial clock line, SCL). In some embodiments, the processor 110 may include a plurality of groups of I2C buses. The processor 110 may be separately coupled to the touch sensor 113A, the power management module 120, and the like through different I2C bus interfaces. For example, the processor 110 may be coupled to the touch sensor 113A through the I2C interface, so that the processor 110 communicates with the touch sensor 113A through the I2C bus interface, to implement a touch function of the electronic device 100.

The I2S interface may be configured to perform audio communication. The PCM interface may also be used to perform audio communication, and sample, quantize, and code an analog signal. The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 111. For example, the processor 110 communicates with a Bluetooth module in the wireless communication module 111 through the UART interface, to implement a Bluetooth function.

The MIPI interface may be configured to connect the processor 110 to a peripheral component like the display 115. The MIPI interface includes a camera serial interface (camera serial interface, CSI), a display serial interface (display serial interface, DSI), and the like. The processor 110 may communicate with the display 115 through the DSI, to implement a display function of the electronic device 100.

The GPIO interface may be configured by software. The GPIO interface may be configured as a control signal or a data signal. The USB interface 119 is an interface that conforms to a USB standard specification, and may be specifically a mini USB interface, a micro USB interface, a USB type-C interface, or the like. The USB interface 119 may be configured to be connected to a charger to charge the electronic device 100, or may be configured to transmit data between the electronic device 100 and a peripheral device.

It may be understood that an interface connection relationship between the modules illustrated in embodiments of the present invention is merely an example for description, and does not constitute a limitation on a structure of the electronic device 100. In some other embodiments of this application, the electronic device 100 may alternatively use an interface connection manner different from that in the foregoing embodiment, or use a combination of a plurality of interface connection manners.

The charging management module 122 is configured to receive a charging input from a charger. The charger may be a wireless charger or a wired charger. In some embodiments of wired charging, the charging management module 122 may receive a charging input of a wired charger through the USB interface 119. In some embodiments of wireless charging, the charging management module 122 may receive a wireless charging input through a wireless charging coil of the electronic device 100. The charging management module 122 may further supply power to the electronic device 100 through the power management module 120 while charging the battery 121.

The power management module 120 is configured to connect to the battery 121, the charging management module 122, and the processor 110. The power management module 120 may receive inputs from the battery 121 and/or the charging management module 122, and supplies power to the processor 110, the internal memory 117, the display 115, the wireless communication module 111, and the like. The power management module 120 may be further configured to monitor parameters such as a battery capacity, a battery cycle count, and a battery health status (electric leakage or impedance). In some other embodiments, the power management module 120 may alternatively be disposed in the processor 110. In some other embodiments, the power management module 120 and the charging management module 122 may alternatively be disposed in a same component.

A wireless communication function of the electronic device 100 may be implemented through the mobile communication module 112, the wireless communication module 111, the modem processor, the baseband processor, and the like.

The mobile communication module 112 may provide a wireless communication solution that is applied to the electronic device 100 and that includes 2G/3G/4G/5G. The mobile communication module 112 may include at least one filter, a switch, a power amplifier, a low noise amplifier (low noise amplifier, LNA), and the like. The mobile communication module 112 may receive an electromagnetic wave through the antenna, perform processing such as filtering and amplification on the received electromagnetic wave, and transmit the electromagnetic wave to the modem processor for demodulation. In some embodiments, at least some functional modules in the mobile communication module 112 may be disposed in the processor 110. In some embodiments, at least some functional modules of the mobile communication module 112 may be disposed in a same component as at least some modules of the processor 110.

The wireless communication module 111 may provide a wireless communication solution that is applied to the electronic device 100 and that includes WLAN (such as a Wi-Fi network), Bluetooth, a global navigation satellite system (global navigation satellite system, GNSS), frequency modulation (frequency modulation, FM), NFC, and an infrared (infrared, IR) technology. The wireless communication module 111 may be one or more components integrating at least one communication processor module. The wireless communication module 111 receives an electromagnetic wave through an antenna, performs frequency modulation and filtering processing on an electromagnetic wave signal, and sends a processed signal to the processor 110. The wireless communication module 111 may further receive a to-be-sent signal from the processor 110, perform frequency modulation and amplification on the signal, and convert the signal into an electromagnetic wave for radiation through the antenna.

The button 114 includes a power button, a volume button, and the like. The button 114 may be a mechanical button, or may be a touch button. The electronic device 100 may receive a button input, and generate a button signal input related to user settings and function control of the electronic device 100.

The display 115 is configured to display an image, a video, and the like. The display 115 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a quantum dot light-emitting diode (quantum dot light-emitting diode, QLED), and the like. In some embodiments, the electronic device 100 may include one or N displays 115, where N is a positive integer greater than 1.

The motor 116 may generate a vibration prompt. The motor 116 may be configured to provide an incoming call vibration prompt and touch vibration feedback. For example, touch operations performed on different applications (for example, photographing and audio playback) may correspond to different vibration feedback effects. The motor 116 may also correspond to different vibration feedback effects for touch operations performed on different areas of the display 115.

The internal memory 117 may be one or more random access memories (random access memory, RAM), and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a 5th generation DDR SDRAM is usually referred to as a DDR5 SDRAM), and the like.

The non-volatile memory may include a magnetic disk storage device and a flash memory (flash memory, FM). The flash memory may be classified into an NOR flash, an NAND flash, a 3D NAND flash, and the like according to an operation principle; may be classified into a single-level cell (single-level cell, SLC), a multi-level cell (multi-level cell, MLC), a triple-level cell (triple-level cell, TLC), a quad-level cell (quad-level cell, QLC), and the like based on a quantity of electric potential levels of a cell; or may be classified into a universal flash storage (universal flash storage, UFS), an embedded multimedia card (embedded multimedia Card, eMMC), and the like according to storage specifications. The random access memory may be directly read and written by the processor 110, may be configured to store executable programs (such as machine instructions) of an operating system or another running program, and may also be configured to store data of users and applications. The non-volatile memory may also store the executable programs, the data of the users and the applications, and the like, and may be loaded into the random access memory in advance, to be directly read and written by the processor 110.

The SIM card interface 118 is configured to connect to a SIM card. The SIM card may be inserted into the SIM card interface 118 or detached from the SIM card interface 118, to implement contact with or separation from the electronic device 100. The electronic device 100 may support one or N SIM card interfaces, where N is a positive integer greater than 1. The SIM card interface 118 may support a nano-SIM card, a micro-SIM card, a SIM card, and the like. A plurality of cards may be inserted into a same SIM card interface 118 at the same time. The plurality of cards may be of a same type or of different types. The SIM card interface 118 is also compatible with different types of SIM cards. The SIM card interface 118 is also compatible with an external memory card. The electronic device 100 interacts with a network by using the SIM card, to implement functions such as conversation and data communication. In some embodiments, the electronic device 100 uses an eSIM, that is, an embedded SIM card. The eSIM card may be embedded into the electronic device 100, and cannot be separated from the electronic device 100.

In some embodiments, the electronic device 100 may alternatively not include the SIM card interface 118.

The touch sensor 113A is also referred to as a "touch component". The touch sensor 113A may be disposed on the display 115, and the touch sensor 113A and the display 115 form a touchscreen, which is also referred to as a "touch screen". The touch sensor 113A is configured to detect a touch operation performed on or near the touch sensor. The touch sensor 113A may transfer the detected touch operation to the application processor to determine a type of a touch event. The touch sensor 113A may provide a visual output related to the touch operation through the display 115. In some other embodiments, the touch sensor 113A may also be disposed on a surface of the electronic device 100 at a location different from that of the display 115. In some embodiments, the electronic device 100 may detect a touch operation on each graphical user interface through the touch sensor 113A, for example, a tap operation on each graphical user interface (for example, a touch operation on a control), and for another example, an upward or downward sliding operation on each graphical user interface.

The barometric pressure sensor 113B is configured to measure barometric pressure. In some embodiments of this application, the electronic device 100 may measure barometric pressure in the airbag 113D through the barometric pressure sensor 113B. In some embodiments of this application, some components of the barometric pressure sensor 113B are located inside the airbag 113D, and are configured to sense air pressure of the airbag 113D.

The micro pump 113C is configured to inflate and deflate air. In some embodiments of this application, the electronic device 100 inflates the airbag 113D through the micro pump 113C, and the micro pump 113C is connected to the airbag 113D through the air path conduction component 113L. The airbag 113D is configured to squeeze a blood vessel of the user. In some embodiments, the electronic device 100 may measure blood pressure and a pulse rate (heart rate) of the user through the barometric pressure sensor 113B, the micro pump 113C, and the airbag 113D.

The magnetic sensor 113E includes a Hall sensor. In some embodiments of this application, the electronic device 100 may determine, through the magnetic sensor 113E, whether the airbag 113D on the electronic device 100 is detached. For example, a magnet may be configured on the airbag 113D or a strap connected to the airbag 113D, and the electronic device 100 may determine, through the magnetic sensor 113E, a magnetic flux generated by the magnet on the airbag 113D or the airbag 113D, and further determine whether the airbag 113D on the electronic device 100 is detached.

The PPG sensor 113F may be configured to collect a PPG signal based on a fluctuation generated by blood vessel contraction, to obtain physiological data of the user. The physiological data includes but is not limited to blood pressure, a heart rate, blood oxygen, a respiratory rate, blood oxygen saturation (SaO2), and the like. In some embodiments, the electronic device 100 may measure a heart rate and a heartbeat interval of the user through the PPG sensor 113F.

The ECG sensor 113H may be configured to collect an ECG signal based on a fluctuation generated by heart contraction, to obtain physiological data of the user. The physiological data includes but is not limited to an electrocardiogram and a heart rate. In some embodiments, the electronic device 100 may measure an electrocardiogram and a heart rate of the user through the ECG sensor 113H.

The gyroscope sensor 113I may be configured to determine a movement posture of the electronic device 100. In some embodiments, angular velocities of the electronic device 100 around three axes (that is, x, y, and z axes) may be determined through the gyroscope sensor 113I, and then the exercise data of the user may be obtained through calculation.

The acceleration sensor 113G may detect magnitudes of accelerations of the electronic device 100 in all directions (generally three axes), and may further obtain the exercise data of the user through calculation. The acceleration sensor 113G may be further configured to identify a posture of the electronic device, and is applied to an application such as a pedometer.

In some embodiments, the electronic device 100 may detect, through the gyroscope sensor 113I, the acceleration sensor 113G, or the like, an exercise gesture performed by the user holding the electronic device 100, for example, shaking the electronic device.

The positioning sensor 113J may be configured to position the electronic device 100, and may include a satellite positioning sensor, for example, a global positioning system (global positioning system, GPS) or BeiDou, which may determine, by receiving a satellite signal, a location of the user wearing the electronic device 100 on the earth. The positioning sensor may also include a communication positioning sensor, for example, a base station or a wireless fidelity (wireless fidelity, Wi-Fi) communication module, which may provide base station positioning, Wi-Fi router positioning, and the like. In addition, the positioning sensor may further include a positioning sensor such as a magnetometer. The magnetometer may measure strength and a direction of a magnetic field, and is configured to determine a direction and a location of the user during the exercise. The exercise may be, for example, a round-trip walking exercise. It is easy to understand that, in some embodiments, the positioning sensor 113J is not mandatory, and is optional.

The timing sensor 113K may be configured to measure time, for example, may calculate exercise duration of the user during the exercise.

The gyroscope sensor 113I, the acceleration sensor 113G, the positioning sensor 113J, and the timing sensor 113K may be collectively referred to as exercise sensors. The exercise sensor may be configured to: collect exercise data, determine an exercise state of the user based on the collected exercise data, and further determine an exercise mode of the user based on the exercise state of the user. It is easy to understand that, in some other embodiments of this application, the exercise sensor may include more or fewer sensors than the foregoing sensors, and different sensor combinations may support different exercise modes.

It should be noted that the air path conduction component 113L may be an independent component, or the air path conduction component 113L may be an air path formed by combining other hardware modules, or the air path conduction component 113L may be a part of another component, for example, may be a part of the micro pump 113C, or may be a part of the airbag 113D.

It should be noted that the sensor module 113 may further include an infrared sensor, a blood oxygen sensor, and the like. The blood oxygen sensor may be configured to detect oxygen partial pressure in blood and convert the oxygen partial pressure into an available output signal. The micro pump 113C may be located inside a watch body of the smartwatch, the airbag 113D may be buckled to a watchband, and the airbag 113D is connected to a watch face through an air hole cover. Correspondingly, the airbag 113D may be separated from the watchband, or may be separated from the watch face.

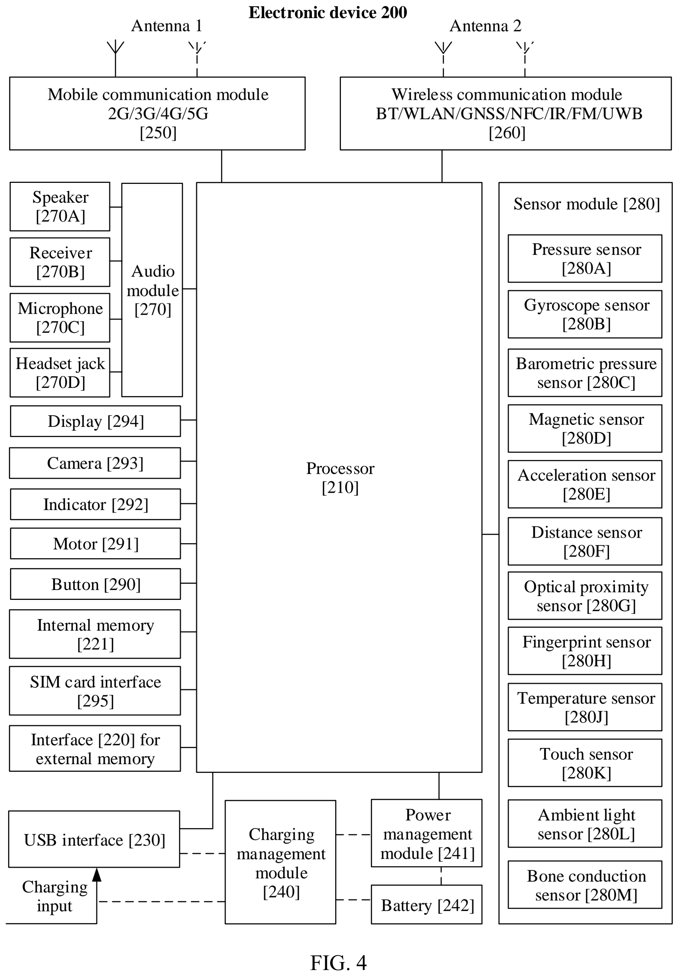

**FIG. 4** **shows an example of an electronic device 200 according to an embodiment of this application.**

The electronic device 200 may be various types of intelligent terminal devices. A specific type of the electronic device 200 is not limited in this embodiment of this application. For example, the electronic device 200 may be a mobile phone, or may be a tablet computer, a desktop computer, a laptop computer, a handheld computer, or a notebook computer.

As shown in FIG. 4, the electronic device 200 may include a processor 210, an interface 220 for external memory, an internal memory 221, a USB interface 230, a charging management module 240, a power management module 241, a battery 242, an antenna 1, an antenna 2, a mobile communication module 250, a wireless communication module 260, an audio module 270, a speaker 270A, a receiver 270B, a microphone 270C, a headset jack 270D, a sensor module 280, a button 290, a motor 291, an indicator 292, a camera 293, a display 294, a SIM card interface 295, and the like. The sensor module 280 may include at least one of a pressure sensor 280A, a gyroscope sensor 280B, a barometric pressure sensor 280C, a magnetic sensor 280D, an acceleration sensor 280E, a distance sensor 280F, an optical proximity sensor 280G, a fingerprint sensor 280H, a temperature sensor 280J, a touch sensor 280K, an ambient light sensor 280L, a bone conduction sensor 280M, and the like.

It may be understood that the structure shown in this embodiment of the present invention does not constitute a specific limitation on the electronic device 200. In some other embodiments of this application, the electronic device 200 may include more or fewer components than those shown in the figure, or a combination of some components, or splits from some components, or a different component layout. The components shown in the figure may be implemented by using hardware, software, or a group of software and hardware.

The processor 210 may include one or more processing units. For example, the processor 210 may include an application processor, a modem processor, a graphics processor, an image signal processor, a controller, a memory, a video codec, a digital signal processor, a baseband processor, and/or a neural network processor. Different processing units may be independent components, or may be integrated into one or more processors. In some embodiments, the electronic device 200 may alternatively include one or more processors 210.

The controller may be a nerve center and a command center of the electronic device 200. The controller may generate an operation control signal based on instruction operation code and a time sequence signal, to complete control of instruction fetching and instruction execution.

The memory may be further disposed in the processor 210, and is configured to store instructions and data. In some embodiments, the memory in the processor 210 is a cache. The memory may store instructions or data just used or cyclically used by the processor 210. If the processor 210 needs to use the instructions or the data again, the processor 210 may directly invoke the instructions or the data from the memory. This avoids repeated access and reduces a waiting time of the processor 210, so that efficiency of the electronic device 200 is improved.

In some embodiments, the processor 210 may include one or more interfaces. The interface may include an I2C interface, an I2S interface, a PCM interface, a UART interface, an MIPI, a GPIO interface, a SIM card interface 295, a USB interface 230, and/or the like.

The I2C interface is a two-way synchronization serial bus, and includes one serial data line and one serial clock line. In some embodiments, the processor 210 may include a plurality of groups of I2C buses. The processor 210 may be separately coupled to the touch sensor 280K, a charger, a flash, the camera 293, and the like through different I2C bus interfaces. For example, the processor 210 may be coupled to the touch sensor 280K through the I2C interface, so that the processor 210 communicates with the touch sensor 280K through the I2C bus interface, to implement a touch function of the electronic device 200.

The I2S interface may be configured to perform audio communication. In some embodiments, the processor 210 may include a plurality of groups of I2S buses. The processor 210 may be coupled to the audio module 270 through the I2S bus, to implement communication between the processor 210 and the audio module 270. In some embodiments, the audio module 270 may transmit an audio signal to the wireless communication module 260 through the I2S interface, to implement a function of answering a call through a Bluetooth headset.

The PCM interface may also be used to perform audio communication, and sample, quantize, and code an analog signal. In some embodiments, the audio module 270 may be coupled to the wireless communication module 260 through a PCM bus interface. In some embodiments, the audio module 270 may alternatively transmit an audio signal to the wireless communication module 260 through the PCM interface, to implement a function of answering a call through a Bluetooth headset. Both the I2S interface and the PCM interface may be configured to perform audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 210 to the wireless communication module 260. For example, the processor 210 communicates with a Bluetooth module in the wireless communication module 260 through the UART interface, to implement a Bluetooth function. In some embodiments, the audio module 270 may transmit an audio signal to the wireless communication module 260 through the UART interface, to implement a function of playing music through a Bluetooth headset.

The MIPI interface may be configured to connect the processor 210 to a peripheral component such as the display 294 or the camera 293. The MIPI interface includes a camera serial interface, a display serial interface, and the like. In some embodiments, the processor 210 communicates with the camera 293 through the CSI, to implement a photographing function of the electronic device 200. The processor 210 communicates with the display 294 through the DSI, to implement a display function of the electronic device 200.

The GPIO interface may be configured by software. The GPIO interface may be configured as a control signal or a data signal. In some embodiments, the GPIO interface may be configured to connect the processor 210 to the camera 293, the display 294, the wireless communication module 260, the audio module 270, the sensor module 280, or the like. The GPIO interface may alternatively be configured as an I2C interface, an I2S interface, a UART interface, an MIPI interface, or the like.

The USB interface 230 is an interface that conforms to a USB standard specification, and may be specifically a mini USB interface, a micro USB interface, a USB Type-C interface, or the like. The USB interface 230 may be configured to connect to a charger to charge the electronic device 200, or may be configured to transmit data between the electronic device 200 and a peripheral device, or may be configured to connect to a headset for playing audio through the headset. The interface may be further configured to connect to another electronic device such as an AR device.

It may be understood that an interface connection relationship between the modules illustrated in this embodiment of the present invention is merely an example for description, and does not constitute a limitation on a structure of the electronic device 200. In some other embodiments, the electronic device 200 may alternatively use an interface connection manner different from that in the foregoing embodiment, or use a combination of a plurality of interface connection manners.

The charging management module 240 is configured to receive a charging input from a charger. The charger may be a wireless charger or a wired charger. In some embodiments of wired charging, the charging management module 240 may receive a charging input of a wired charger through the USB interface 230. In some embodiments of wireless charging, the charging management module 240 may receive a wireless charging input through a wireless charging coil of the electronic device 200. The charging management module 240 supplies power to the electronic device through the power management module 241 while charging the battery 242.

The power management module 241 is configured to connect the battery 242 and the charging management module 240 to the processor 210. The power management module 241 receives an input from the battery 242 and/or the charging management module 240, and supplies power to the processor 210, the internal memory 221, an external memory, the display 294, the camera 293, the wireless communication module 260, and the like. The power management module 241 may be further configured to monitor parameters such as a battery capacity, a battery cycle count, and a battery health status (electric leakage or impedance). In some other embodiments, the power management module 241 may alternatively be disposed in the processor 210. In some other embodiments, the power management module 241 and the charging management module 240 may alternatively be disposed in a same component.

A wireless communication function of the electronic device 100 may be implemented by using the antenna 1, the antenna 2, the mobile communication module 250, the wireless communication module 260, the modem processor, the baseband processor, and the like.

The antenna 1 and the antenna 2 are configured to transmit and receive electromagnetic wave signals. Each antenna in the electronic device 200 may be configured to cover one or more communication frequency bands. Different antennas may be further reused, to improve antenna utilization. For example, the antenna 1 may be reused as a diversity antenna of a wireless local area network. In some other embodiments, the antenna may be used in combination with a tuning switch.

The mobile communication module 250 may provide a wireless communication solution that is applied to the electronic device 200 and that includes 2G/3G/4G/5G. The mobile communication module 250 may include at least one filter, a switch, a power amplifier, a low noise amplifier, and the like. The mobile communication module 250 may receive an electromagnetic wave through the antenna 1, perform processing such as filtering and amplification on the received electromagnetic wave, and transmit the electromagnetic wave to the modem processor for demodulation. The mobile communication module 250 may further amplify a signal modulated by the modem processor, and convert the signal into an electromagnetic wave for radiation through the antenna. In some embodiments, at least some functional modules in the mobile communication module 250 may be disposed on the processor 210. In some embodiments, at least some functional modules in the mobile communication module 250 may be disposed on a same component as at least some modules in the processor 210.

The modem processor may include a modulator and a demodulator. The modulator is configured to modulate a to-be-transmitted low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 270A, the receiver 270B, or the like), or displays an image or a video through the display 294. In some embodiments, the modem processor may be an independent component. In some other embodiments, the modem processor may be independent of the processor 210, and is disposed in a same component as the mobile communication module 250 or another functional module.

The wireless communication module 260 may provide a wireless communication solution that is applied to the electronic device 200 and that includes WLAN (such as a Wi-Fi network), Bluetooth, a global navigation satellite system, frequency modulation, NFC, an infrared technology, and an ultra wide band (ultra wide band, UWB). The wireless communication module 260 may be one or more components integrating at least one communication processor module. The wireless communication module 260 receives an electromagnetic wave through an antenna, performs frequency modulation and filtering processing on an electromagnetic wave signal, and sends a processed signal to the processor 210. The wireless communication module 260 may further receive a to-be-sent signal from the processor 210, perform frequency modulation and amplification on the signal, and convert the signal into an electromagnetic wave for radiation through the antenna. For example, the wireless communication module 260 may include a Bluetooth module, a Wi-Fi module, and the like.

In some embodiments, some antennas of the electronic device 200 are coupled to the mobile communication module 250, and the other antennas are coupled to the wireless communication module 260, so that the electronic device 200 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), millimeter wave (millimeter wave, mmWave), BT, a GNSS, a WLAN, NFC, FM, UWB, an IR technology, and/or the like. The GNSS may include a GPS, a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation systems, SBAS).

The electronic device 200 may implement a display function through the GPU, the display 294, the application processor, and the like, for example, may display an exercise and health result report of the user. The GPU is a microprocessor for image processing, and is connected to the display 294 and the application processor. The GPU is configured to: perform mathematical and geometric computation, and render an image. The processor 210 may include one or more GPUs, which execute instructions to generate or change display information.

The display 294 is configured to display an image, a video, or the like. The display 294 includes a display panel. The display panel may be an LCD, an OLED, an active-matrix organic light-emitting diode, an AMOLED, an FLED, a mini-LED, a micro-LED, a micro-OLED, a QLED, or the like. In some embodiments, the electronic device 200 may include one or N display screens 294, where N is a positive integer greater than 1.

In some embodiments, the display 294 of the electronic device 200 is larger than the display 115 of the electronic device 100.

The electronic device 200 may implement a photographing function through the ISP, the camera 293, the video codec, the GPU, the display 294, the application processor, and the like.

The ISP is configured to process data fed back by the camera 293. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and color of the image. The ISP may further optimize parameters such as exposure and a color temperature of a photographing scenario. In some embodiments, the ISP may be disposed in the camera 293.

The camera 293 is configured to capture a static image or a video. An optical image of an object is generated through a lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format such as RGB or YUV. In some embodiments, the electronic device 200 may include one or N cameras 293, where N is a positive integer greater than 1.

The digital signal processor is configured to process a digital signal, and may process another digital signal in addition to the digital image signal. For example, when the electronic device 200 selects a frequency, the digital signal processor is configured to perform Fourier transformation on frequency energy.

The video codec is configured to compress or decompress a digital video. The electronic device 200 may support one or more video codecs. In this way, the electronic device 200 may play or record videos in a plurality of encoding formats, for example, moving picture experts group (moving picture experts group, MPEG)-1, MPEG-2, MPEG-3, and MPEG-4.

The NPU is a neural-network (neural-network, NN) computing processor. The NPU quickly processes input information by referring to a structure of a biological neural network, for example, a transfer mode between human brain neurons, and may further continuously perform self-learning. Applications such as intelligent cognition of the electronic device 200 may be implemented through the NPU, for example, image recognition, facial recognition, speech recognition, and text understanding.

The interface 220 for external memory may be used to connect to an external memory card, for example, a micro SD card, to extend a storage capability of the electronic device 200. The external memory card communicates with the processor 210 through the interface 220 for external memory, to implement a data storage function. For example, data such as music, a photo, or a video is stored in the external memory card.

The internal memory 221 may be configured to store one or more computer programs. The one or more computer programs include instructions. The processor 210 may run the instructions stored in the internal memory 221, so that the electronic device 200 performs a data sharing method provided in some embodiments of this application, various functional applications, data processing, and the like. The internal memory 221 may include a program storage area and a data storage area. The program storage area may store an operating system. The program storage area may further store one or more applications (such as Gallery or Contacts), and the like. The data storage area may store data (for example, a photo or a contact) created in a process of using the electronic device 200. In addition, the internal memory 221 may include a high-speed random access memory, or may include a nonvolatile memory, for example, at least one magnetic disk storage device, a flash memory, or a universal flash storage (universal flash storage, UFS).

The electronic device 200 may implement an audio function such as music playing or recording through the audio module 270, the speaker 270A, the receiver 270B, the microphone 270C, the headset jack 270D, the application processor, and the like.

The audio module 270 is configured to convert digital audio information into an analog audio signal for output, and is also configured to convert an analog audio input into a digital audio signal. The audio module 270 may be further configured to encode and decode an audio signal. In some embodiments, the audio module 270 may be disposed in the processor 210, or some functional modules of the audio module 270 are disposed in the processor 210.

The speaker 270A, also referred to as a "loudspeaker", is configured to convert an audio electrical signal into a sound signal. The electronic device 200 may be used to listen to music or answer a call in a hands-free mode over the speaker 270A.

The receiver 270B, also referred to as an "earpiece", is configured to convert an electrical audio signal into a sound signal. When a call is answered or a voice message is received through the electronic device 200, the receiver 270B may be put close to a human ear to listen to a voice.

The microphone 270C, also referred to as a "mike" or a "mic", is configured to convert a sound signal into an electrical signal. When making a call or sending voice information, the user may make a sound near the microphone 270C through the mouth of the user, to enter a sound signal to the microphone 270C. At least one microphone 270C may be disposed in the electronic device 200. In some other embodiments, two microphones 270C may be disposed in the electronic device 200, to collect a sound signal and implement a noise reduction function. In some other embodiments, three, four, or more microphones 270C may alternatively be disposed in the electronic device 200, to collect a sound signal, implement noise reduction, and identify a sound source, to implement a directional recording function and the like.

The headset jack 270D is configured to connect to a wired headset. The headset jack 270D may be the USB interface 230, or may be a 3.5 mm open mobile terminal platform (open mobile terminal platform, OMTP) standard interface, or a cellular telecommunications industry association of the USA (cellular telecommunications industry association of the USA, CTIA) standard interface.

The pressure sensor 280A is configured to sense a pressure signal, and can convert the pressure signal into an electrical signal. In some embodiments, the pressure sensor 280A may be disposed on the display 294. There are a plurality of types of pressure sensors 280A, such as a resistive pressure sensor, an inductive pressure sensor, and a capacitive pressure sensor. The capacitive pressure sensor may include at least two parallel plates made of conductive materials. When a force is applied to the pressure sensor 280A, capacitance between electrodes changes. The electronic device 200 determines pressure intensity based on the change of the capacitance. When a touch operation is performed on the display 294, the electronic device 200 detects intensity of the touch operation through the pressure sensor 280A. The electronic device 200 may also calculate a touch location based on a detection signal of the pressure sensor 280A. In some embodiments, touch operations that are performed in a same touch location but have different touch operation intensity may correspond to different operation instructions. For example, when a touch operation whose touch operation intensity is less than a first pressure threshold is performed on an SMS message application icon, an instruction for viewing an SMS message is performed. When a touch operation whose touch operation intensity is greater than or equal to the first pressure threshold is performed on the SMS message application icon, an instruction for creating a new SMS message is performed.

The gyroscope sensor 280B may be configured to determine a movement posture of the electronic device 200. In some embodiments, angular velocities of the electronic device 200 around three axes (namely, axes x, y, and z) may be determined through the gyroscope sensor 280B. The gyroscope sensor 280B may be configured to implement image stabilization during photographing. For example, when the shutter is pressed, the gyroscope sensor 280B detects an angle at which the electronic device 200 jitters, calculates, based on the angle, a distance for which a lens module needs to compensate, and allows the lens to cancel the jitter of the electronic device 200 through reverse motion, to implement image stabilization. The gyroscope sensor 280B may also be used in a navigation scenario or a somatic game scenario.

The barometric pressure sensor 280C is configured to measure barometric pressure. In some embodiments, the electronic device 200 calculates an altitude through the barometric pressure measured by the barometric pressure sensor 280C, to assist in positioning and navigation.

The magnetic sensor 280D includes a Hall sensor. The electronic device 200 may detect opening and closing of a flip cover by using the magnetic sensor 280D. In some embodiments, when the electronic device 200 is a clamshell phone, the electronic device 200 may detect opening and closing of a flip cover based on the magnetic sensor 280D. Further, a feature like automatic unlocking upon opening of the flip cover is set based on a detected opening or closing state of the flip cover.

The acceleration sensor 280E may detect magnitude of acceleration of the electronic device 200 in various directions (usually on three axes). Magnitude and a direction of gravity may be detected when the electronic device 200 is static. The acceleration sensor 280E may be further configured to identify a posture of the electronic device, and is used in switching between a landscape mode and a portrait mode or an application like a pedometer.

The distance sensor 280F is configured to measure a distance. The electronic device 200 may measure a distance through infrared or laser. In some embodiments, in a photographing scenario, the electronic device 200 may measure a distance by using the distance sensor 280F to implement quick focusing.

The optical proximity sensor 280G may include, for example, a light-emitting diode (light-emitting diode, LED) and an optical detector, for example, a photodiode. The light-emitting diode may be an infrared light-emitting diode. The electronic device 200 emits infrared light by using the light emitting diode. The electronic device 200 detects infrared reflected light from a nearby object by using the photodiode. When sufficient reflected light is detected, it may be determined that there is an object near the electronic device 200. When insufficient reflected light is detected, the electronic device 200 may determine that there is no object near the electronic device 200. The electronic device 200 may detect, by using the optical proximity sensor 280G, that the user holds the electronic device 200 close to an ear for a call, to automatically turn off a screen for power saving. The optical proximity sensor 280G may also be used in a smart cover mode or a pocket mode to automatically perform screen unlocking or locking.

The ambient light sensor 280L is configured to sense ambient light brightness. The electronic device 200 may adaptively adjust brightness of the display 294 based on the sensed ambient light brightness. The ambient light sensor 280L may also be configured to automatically adjust white balance during photographing. The ambient light sensor 280L may also cooperate with the optical proximity sensor 280G to detect whether the electronic device 200 is in a pocket, to avoid an accidental touch.

The fingerprint sensor 280H is configured to collect a fingerprint. The electronic device 200 may use a feature of the collected fingerprint to implement fingerprint-based unlocking, application lock access, fingerprint-based photographing, fingerprint-based call answering, and the like.

The temperature sensor 280J is configured to detect a temperature. In some embodiments, the electronic device 200 executes a temperature processing policy based on the temperature detected by the temperature sensor 280J. For example, when the temperature reported by the temperature sensor 280J exceeds a threshold, the electronic device 200 lowers performance of a processor located near the temperature sensor 280J, to reduce power consumption for thermal protection. In some other embodiments, when the temperature is lower than another threshold, the electronic device 200 heats the battery 242 to prevent the electronic device 200 from being shut down abnormally due to a low temperature. In some other embodiments, when the temperature is lower than still another threshold, the electronic device 200 boosts an output voltage of the battery 242 to avoid abnormal shutdown caused by a low temperature.

The touch sensor 280K may also be referred to as a touch panel or a touch-sensitive surface. The touch sensor 280K may be disposed on the display 294. The touch sensor 280K and the display 294 form a touchscreen, which is also referred to as a "touch screen". The touch sensor 280K is configured to detect a touch operation performed on or near the touch sensor. The touch sensor may transfer the detected touch operation to the application processor to determine a type of a touch event. A visual output related to the touch operation may be provided through the display 294. In some other embodiments, the touch sensor 280K may also be disposed on a surface of the electronic device 200 at a location different from that of the display 294.

The bone conduction sensor 280M may obtain a vibration signal. In some embodiments, the bone conduction sensor 280M may obtain a vibration signal of a vibration bone of a human vocal-cord part. The bone conduction sensor 280M may also be in contact with a body pulse to receive a blood pressure beating signal. In some embodiments, the bone conduction sensor 280M may also be disposed in a headset, to obtain a bone conduction headset. The audio module 270 may obtain a speech signal through parsing based on the vibration signal that is of the vibration bone of the vocal-cord part and that is obtained by the bone conduction sensor 280M, to implement a speech function. The application processor may parse heart rate information based on the blood pressure beating signal obtained by the bone conduction sensor 280M, to implement a heart rate detection function.

The button 290 includes a power button, a volume button, and the like. The button 290 may be a mechanical button, or may be a touch button. The electronic device 200 may receive a key input, and generate a key signal input related to a user setting and function control of the electronic device 200.

The motor 291 may generate a vibration prompt. The motor 291 may be configured to provide an incoming call vibration prompt and touch vibration feedback. For example, touch operations performed on different applications (for example, photographing and audio playback) may correspond to different vibration feedback effects. The motor 291 may also correspond to different vibration feedback effect for touch operations performed on different areas of the display 294. Different application scenarios (for example, a time reminder, information receiving, an alarm clock, and a game) may also correspond to different vibration feedback effects. A touch vibration feedback effect may be further customized.

The indicator 292 may be an indicator light, and may be configured to indicate a charging status and a power change, or may be configured to indicate a message, a missed call, a notification, and the like.

The SIM card interface 295 is configured to connect to a SIM card. The SIM card may be inserted into the SIM card interface 295 or detached from the SIM card interface 295, to implement contact with or separation from the electronic device 200. The electronic device 200 may support one or N SIM card interfaces, where N is a positive integer greater than 1. The SIM card interface 295 may support a nano-SIM card, a micro-SIM card, a SIM card, and the like. A plurality of cards may be inserted into a same SIM card interface 295 at the same time. The plurality of cards may be of a same type or different types. The SIM card interface 295 is also compatible with different types of SIM cards. The SIM card interface 295 is also compatible with an external memory card. The electronic device 200 interacts with a network by using the SIM card, to implement functions such as conversation and data communication. In some embodiments, the electronic device 200 uses an eSIM, that is, an embedded SIM card. The eSIM card may be embedded into the electronic device 200, and cannot be separated from the electronic device 200.

**FIG. 5** **shows an overall procedure of a detection method according to an embodiment of this application.** The method may be applied to the communication system shown in FIG. 2.

As shown in FIG. 5, the method may include the following steps.

**S501: An electronic device 100 collects exercise-related physiological data.**

Specifically, the exercise-related physiological data may include one or more of the following: pre-exercise physiological data, in-exercise physiological data, and post-exercise physiological data.

Pre-exercise may be a period before a user starts an exercise, and the user is in a resting state in the period. In-exercise may be a period in which the user is performing the exercise or pauses the exercise. Post-exercise may be a period within preset duration (for example, within 10 minutes) after the exercise of the user ends.

How to collect exercise-related physiological data is described in detail in the following content, and details are not described herein.

**S502: The electronic device 100 obtains, through analysis, an exercise and health result of the user based on the collected exercise-related physiological data, and displays the exercise and health result.**

In this embodiment of this application, the exercise and health result (which may also be referred to as an exercise and health status) may be a physical health status of the user at one or more of a pre-exercise stage, an in-exercise stage, and a post-exercise stage, for example, a cardiovascular health status. The physical health status is determined based on the exercise-related physiological data collected in step S501.

Specifically, the exercise and health result may include a blood pressure evaluation result and cardiovascular risk prediction. The blood pressure evaluation result may indicate a blood pressure level of the user at one or more of the pre-exercise stage, the in-exercise stage, and the post-exercise stage. The blood pressure evaluation result may be generated based on the pre-exercise physiological data, the in-exercise physiological data, and the post-exercise physiological data, or generated based on the pre-exercise physiological data and the post-exercise physiological data. The blood pressure evaluation result may include blood pressure response evaluation and a blood pressure control level. The blood pressure response evaluation indicates a blood pressure level evaluation result of the user in an entire exercise process. The blood pressure control level indicates a blood pressure level change status of the user in the entire exercise process. The cardiovascular risk prediction may indicate an exercise-related cardiovascular risk evaluation result of the user. The cardiovascular risk prediction may be generated based on two or three of the pre-exercise physiological data, the in-exercise physiological data, and the post-exercise physiological data, and one or more of an exercise mode, a pre-exercise questionnaire, and a post-exercise questionnaire.

In some embodiments, the exercise and health result may further include first evaluation. The first evaluation may indicate an exercise suggestion for the user during the exercise, and the exercise suggestion may indicate an exercise state that is suitable for or not suitable for the user in different exercise states.

The following describes in detail how the electronic device 100 obtains the exercise-related physiological data, how to obtain, through analysis, the exercise and health result of the user based on the obtained exercise-related physiological data, and how to present the exercise and health result.

### Collection of exercise-related physiological data

### Collection of pre-exercise physiological data

The pre-exercise physiological data may include one or more of the following: blood pressure and a heart rate. The blood pressure may be collected by using a barometric pressure sensor, a micro pump, and an airbag, and the heart rate may be collected by using a PPG sensor.

Manner 1: Collection of the pre-exercise physiological data is triggered based on a user input.

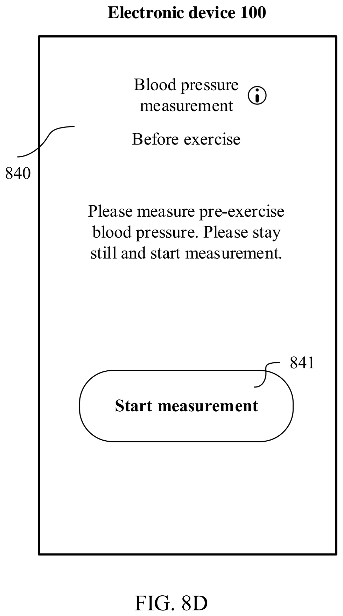

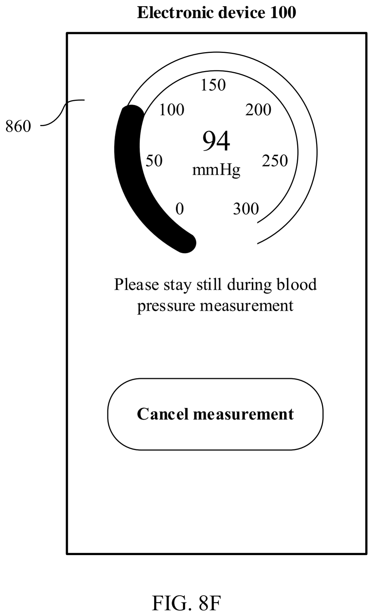

The electronic device 100 may display a first user interface, for example, a user interface 840 shown in FIG. 8D, and detect a first user operation performed by the user on the first user interface, for example, an operation of tapping an option 841. In response to the operation, the electronic device 100 may start to collect the pre-exercise physiological data. This manner is applicable to physiological data that needs to be collected when the user is still, for example, blood pressure. To ensure that the user is in a still state, the electronic device 100 may display a user interface 860 shown in FIG. 8F, to prompt the user to stay still when such physiological data (for example, blood pressure) is measured.

The pre-exercise physiological data is collected in Manner 1, and the electronic device 100 does not need to collect the physiological data a plurality of times when detecting that the user does not exercise, so that power consumption can be reduced.

Manner 2: The pre-exercise physiological data is collected when it is detected that the user is in a non-exercise state.

Alternatively, the electronic device 100 may collect physiological data when detecting that the user does not exercise, further determine, with reference to the collected exercise data, when the user is in pre-exercise, and use the physiological data as the pre-exercise physiological data. Further, the electronic device 100 may collect physiological data a plurality of times when detecting that the user does not exercise, and use pre-exercise physiological data collected most recently as the pre-exercise physiological data.

In some embodiments, the electronic device 100 may not collect the pre-exercise physiological data, but use historical pre-exercise physiological data as the pre-exercise physiological data. The historical pre-exercise physiological data may be blood pressure and heart rate data of the user in a resting state in a recent period of time (for example, within one week), for example, a 24-hour average blood pressure value and a 24-hour average heart rate value.

How to determine, based on the collected exercise data, when the user is in pre-exercise is described in detail in the following content, and details are not described herein.

### Collection of in-exercise physiological data

The in-exercise physiological data may include one or more of the following: a heart rate, blood pressure, an electrocardiogram, and blood oxygen. The heart rate may be collected by using a PPG sensor, the blood pressure may be collected by using a barometric pressure sensor, a micro pump, and an airbag, the electrocardiogram may be collected by using an ECG sensor, and the blood oxygen may be collected by using a blood oxygen sensor.

Manner 1: Collection of the in-exercise physiological data is triggered based on a user input.

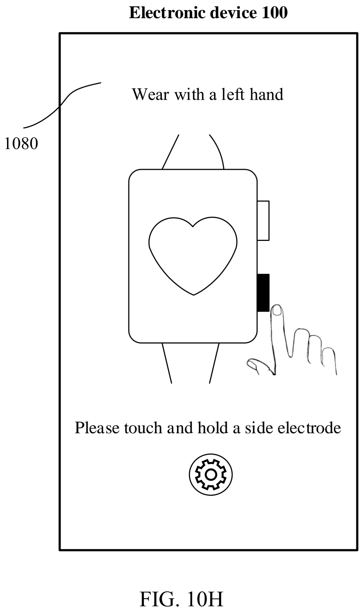

The electronic device 100 may display a second user interface, for example, a user interface 920 shown in FIG. 9B, and detect a second user operation performed by the user on the second user interface, for example, an operation of tapping an option 921 or an operation of tapping an option 922. In response to the operation, the electronic device 100 may start to collect the in-exercise physiological data. Specifically, the second user interface may be displayed when the user pauses the exercise. For example, when it is detected that the user taps a control 911 on the user interface 910 shown in FIG. 9A, it is determined that the user pauses the exercise, and the electronic device 100 may display the user interface 920 shown in FIG. 9B. This manner is applicable to physiological data that needs to be collected when the user is still, for example, blood pressure and an electrocardiogram. To ensure that the user is in a still state, the electronic device 100 may display a user interface 1080 shown in FIG. 10H, to prompt the user to stay still when such physiological data (for example, an electrocardiogram) is measured.

Manner 2: The in-exercise physiological data is collected when it is detected that the user is in an exercise state.

Alternatively, the electronic device 100 may collect physiological data when detecting that the user is in the exercise state, further determine, with reference to the collected exercise data, when the user is in in-exercise, and use the physiological data as the in-exercise physiological data.

Collection of the in-exercise physiological data in Manner 2 may be imperceptible to the user. To be specific, the electronic device 100 may continuously collect physiological data such as a heart rate, blood pressure, and blood oxygen when the user is in in-exercise, without a need to be triggered by the user. Continuous collection may include uninterrupted collection and a plurality of collections at a specific time interval (for example, 2 minutes).

How to determine, based on the collected exercise data, when the user is in in-exercise is described in detail in the following content, and details are not described herein.

### Collection of post-exercise physiological data

The post-exercise physiological data may include one or more of the following: a heart rate, blood pressure, an electrocardiogram, and blood oxygen. The heart rate may be collected by using a PPG sensor, the blood pressure may be collected by using a barometric pressure sensor, a micro pump, and an airbag, the electrocardiogram may be collected by using an ECG sensor, and the blood oxygen may be collected by using a blood oxygen sensor.

Manner 1: Collection of the post-exercise physiological data is triggered based on a user input.

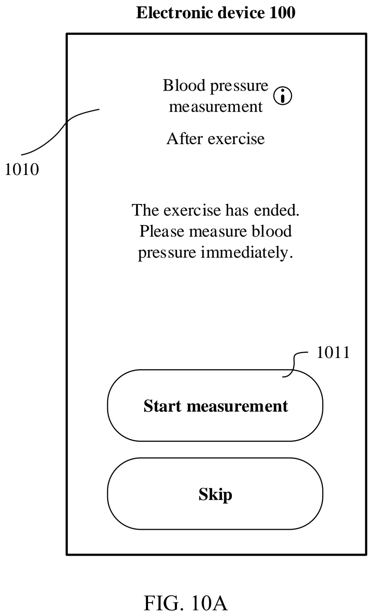

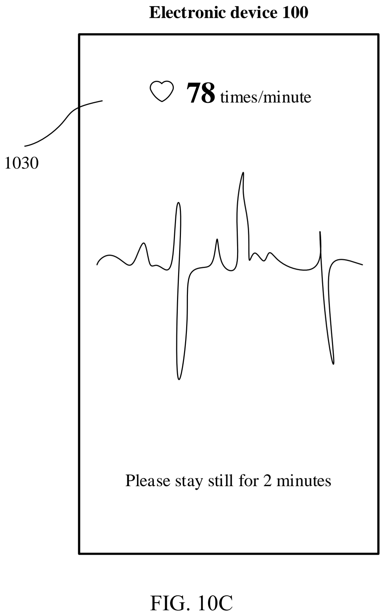

The electronic device 100 may display a third user interface, for example, a user interface 1010 shown in FIG. 10A, and detect a third user operation performed by the user on the third user interface, for example, an operation of tapping an option 1011. In response to the operation, the electronic device 100 may start to collect the post-exercise physiological data. Specifically, the third user interface may be displayed when the user ends the exercise. For example, when it is detected that the user taps a control 912 on the user interface 910 shown in FIG. 9A, it is determined that the user ends the exercise, and the electronic device 100 may display the user interface 1010 shown in FIG. 10A. This manner is applicable to physiological data that needs to be collected when the user is still, for example, blood pressure, a heart rate, and an electrocardiogram. To ensure that the user is in a still state, the electronic device 100 may display a user interface 1030 shown in FIG. 10C, to prompt the user to stay still when such physiological data (for example, a heart rate) is measured.

Manner 2: The post-exercise physiological data is collected when it is detected that the exercise of the user ends.

Alternatively, the electronic device 100 may collect physiological data within preset duration (for example, within 10 minutes) after detecting that the exercise of the user ends, further determine, with reference to the collected exercise data, when the user is in post-exercise, and use the physiological data as the post-exercise physiological data.

Collection of the post-exercise physiological data in Manner 2 may be imperceptible to the user. To be specific, the electronic device 100 may continuously collect physiological data such as a heart rate, blood pressure, and blood oxygen when the user is in post-exercise, without a need to be triggered by the user. Continuous collection may include uninterrupted collection and a plurality of collections at a specific time interval (for example, 2 minutes).

In some embodiments, collection of electrocardiogram data may alternatively be imperceptible to the user, but accuracy of electrocardiogram data collected in a manner imperceptible to the user is lower than accuracy of electrocardiogram data collected in a manner perceptible to the user.

How to determine, based on the collected exercise data, when the user is in post-exercise is described in detail in the following content, and details are not described herein.

### Analysis of exercise and health result

In this embodiment of this application, there may be a plurality of algorithm models for analyzing the exercise and health result, and different exercise modes correspond to different algorithm models. To more accurately analyze the exercise and health result of the user, the electronic device 100 may select, based on an exercise mode of the user, an algorithm model used to analyze the exercise and health result.

In this embodiment of this application, the exercise mode may also be referred to as an exercise type, and may include but is not limited to the following exercise modes: indoor walking, outdoor walking, indoor running, outdoor running, round-trip walking, an elliptical machine, and a rowing machine. The exercise mode may alternatively be user-defined, for example, a "six-minute walking" exercise mode.

In some embodiments, the exercise mode may be specified by the user. For example, on a user interface 820 shown in FIG. 8B displayed by the electronic device 100, the user may tap an icon 822 to select the exercise mode as "outdoor running".

In some other embodiments, the exercise mode may be automatically identified by the electronic device 100 based on collected exercise data such as an exercise speed, an exercise distance, exercise duration, an exercise direction, a location, an altitude, a pace, a step count, and a metabolic equivalent of task. For example, the electronic device 100 may determine, based on exercise data collected by a gyroscope sensor and an acceleration sensor, that a user is running, and then may further determine, with reference to location data collected by a positioning sensor, that the user is running outdoors. Therefore, the electronic device 100 can identify that an exercise mode is "outdoor running". For another example, the electronic device 100 may determine, based on exercise data collected by a positioning sensor like a magnetometer and an acceleration sensor, that a user moves back and forth at a low speed. Therefore, the electronic device 100 can identify that an exercise mode is "round-trip walking".

In this embodiment of this application, exercise-related physiological data in different ranges is input into an algorithm model corresponding to an exercise mode, and different exercise and health results may be obtained through processing. The exercise and health results may be reflected as a cardiovascular health status, and the cardiovascular health status may be evaluated based on a cardiovascular risk evaluation result. For example, a cardiovascular health status corresponding to an exercise and health result being excellent is excellent, and a cardiovascular risk evaluation result corresponding to the cardiovascular health status being excellent is low risk; a cardiovascular health status corresponding to the exercise and health result being good is good, and a cardiovascular risk evaluation result corresponding to the cardiovascular health status being good is medium risk; and a cardiovascular health status corresponding to an exercise and health result being poor is poor, and a cardiovascular risk evaluation result corresponding to the cardiovascular health status being poor is high risk.

For example, for an algorithm model corresponding to an exercise mode like outdoor running, when a blood pressure value (a blood pressure value measured during the exercise or a blood pressure increase value compared with that before the exercise) is within a first range, a corresponding cardiovascular risk evaluation result is low risk, that is, a corresponding exercise and health result is excellent; or when a blood pressure value (a blood pressure value measured during the exercise or a blood pressure increase value compared with that before the exercise) is within a second range, a corresponding cardiovascular risk evaluation result is medium risk, that is, a corresponding exercise and health result is good; or when a blood pressure value (a blood pressure value measured during the exercise or a blood pressure increase value compared with that before the exercise) is within a third range, a corresponding cardiovascular risk evaluation result is high risk, that is, a corresponding exercise and health result is poor.

**FIG. 6** **shows an example of selecting an algorithm model corresponding to an exercise mode to analyze an exercise and health result.**

As shown in FIG. 6, the algorithm model for analyzing the exercise and health result may include an algorithm model 1, an algorithm model 2, ..., and an algorithm model m, where m is a positive integer greater than 2. The algorithm model 1, the algorithm model 2, ..., and the algorithm model m may be respectively algorithm models corresponding to exercise modes such as "indoor cycling", "outdoor running", ..., and "elliptical machine". If the electronic device 100 determines that the exercise mode is "outdoor running", the electronic device 100 may analyze the physiological data and the exercise data by using the algorithm model 2, to obtain an exercise and health result of outdoor running.

Specifically, the algorithm model used to analyze the exercise and health result may be a neural network model, and may be obtained through training based on a first training set. A confidence level may be higher than a specific confidence value, for example, 90%. The first training set includes a large quantity of training samples. One of these training samples may include an input and an output sample corresponding to the input. The input may include exercise data and physiological data obtained when the exercise data is collected. The output sample may be an exercise and health result. The exercise and health result may generally be a health condition that may be recognized in the medical field when a person performs the exercise indicated by the exercise data in the training sample and physiological data like blood pressure, a heart rate, an electrocardiogram, and blood oxygen of the person resembles the physiological data in the training sample. Therefore, the exercise and health result is usually trustworthy and can be used to train a neural network.

Further, algorithm models may be separately set for three stages: pre-exercise, in-exercise, and an entire exercise process (including pre-exercise, in-exercise, and post-exercise), to output exercise and health suggestions or exercise and health results of different stages.

**FIG. 7A** **shows a method for providing an exercise and health suggestion based on an algorithm model corresponding to pre-exercise.**

As shown in FIG. 7A, an algorithm model X is a model used to provide a pre-exercise exercise and health suggestion based on historical physiological data and historical exercise data. In other words, an input of the algorithm model X includes a first input and a second input. The first input is the historical physiological data, and the second input is the historical exercise data. An output of the algorithm model X is the pre-exercise exercise and health suggestion. For example, the pre-exercise exercise and health suggestion like a recommended exercise mode, recommended exercise duration, or risk warning may be output. In this way, the algorithm model X can provide the pre-exercise exercise and health suggestion before the exercise of the user, to better help the user prevent an exercise risk. In addition, input and calibration operations of the user can be reduced, and the user does not need to manually enter data, enhancing user experience.

Specifically, the algorithm model X used to provide the pre-exercise exercise and health suggestion may be a neural network model, and may be obtained through training based on a second training set. A confidence level may be higher than a specific confidence value, for example, 90%. The second training set includes a large quantity of training samples. One of these training samples may include an input and an output sample corresponding to the input. The input may include historical exercise data and historical physiological data obtained when the historical exercise data is collected. The output sample may be a pre-exercise exercise and health suggestion. The pre-exercise exercise and health suggestion may generally be an exercise and health suggestion that may be given by a medical expert when a person performs the exercise indicated by the historical exercise data in the training sample and physiological data like blood pressure, a heart rate, an electrocardiogram, and blood oxygen of the person resembles the historical physiological data in the training sample. Therefore, the pre-exercise exercise and health suggestion is usually trustworthy and can be used to train a neural network.

The historical physiological data may include pre-exercise physiological data, in-exercise physiological data, and post-exercise physiological data of a previous exercise of the user. The historical exercise data may include exercise data of the previous exercise. The pre-exercise exercise and health suggestion may include a pre-exercise exercise suggestion and a pre-exercise health suggestion. The pre-exercise exercise suggestion may indicate whether the user is suitable for a current exercise mode, and the pre-exercise health suggestion may indicate physiological data that the user needs to pay attention to in a current exercise process.

**FIG. 7B** **shows a method for providing an exercise and health suggestion based on an algorithm model corresponding to in-exercise.**

As shown in FIG. 7B, an algorithm model Y is a model used to provide an in-exercise exercise and health suggestion based on in-exercise physiological data and exercise data. In other words, an input of the algorithm model Y includes a third input and a fourth input. The third input is the in-exercise physiological data, and the fourth input is the exercise data. An output of the algorithm model Y is the in-exercise exercise and health suggestion. For example, the in-exercise exercise and health suggestion like continuing exercise, reducing exercise intensity, increasing exercise intensity, stopping exercise, continuing exercise after resting for preset duration (for example, five minutes), and continuing exercise after resting for preset duration (for example, five minutes) and no abnormality is detected may be output. In this way, the algorithm model Y can provide a real-time exercise and health suggestion during the exercise of the user, so that real-time exercise safety monitoring of the user can be better implemented, and the user can know a potential risk in time and a suggestion or a warning is provided during the exercise, to avoid occurrence of a cardiovascular risk event caused by excessive exercise intensity of the user.

Specifically, the algorithm model Y used to provide the in-exercise exercise and health suggestion may be a neural network model, and may be obtained through training based on a third training set. A confidence level may be higher than a specific confidence value, for example, 90%. The third training set includes a large quantity of training samples. One of these training samples may include an input and an output sample corresponding to the input. The input may include exercise data and in-exercise physiological data obtained when the exercise data is collected. The output sample may be an in-exercise exercise and health suggestion. The in-exercise exercise and health suggestion may generally be an exercise and health suggestion that may be given by a medical expert when a person performs the exercise indicated by the exercise data in the training sample and physiological data like blood pressure, a heart rate, an electrocardiogram, and blood oxygen of the person resembles the in-exercise physiological data in the training sample. Therefore, the in-exercise exercise and health suggestion is usually trustworthy and can be used to train a neural network.

In some embodiments, the electronic device 100 may obtain, through analysis based on the in-exercise physiological data and the exercise data, exercise and health results when the user is in different exercise states during the exercise, to obtain the in-exercise exercise and health suggestion. The in-exercise exercise and health suggestion may include an in-exercise exercise suggestion and an in-exercise health suggestion. The in-exercise exercise suggestion may indicate an exercise state that is suitable or unsuitable for the user in different exercise states, and the in-exercise health suggestion may indicate abnormal physiological data in a current exercise process.

**FIG. 7C** **shows a method for obtaining an exercise and health result based on an algorithm model corresponding to an entire exercise process.**

As shown in FIG. 7C, an algorithm model Z is a model used to provide an exercise and health result based on pre-exercise physiological data, in-exercise physiological data, and post-exercise physiological data. In other words, an input of the algorithm model Z includes a fifth input, a sixth input, and a seventh input. The fifth input is pre-exercise physiological data, the sixth input is in-exercise physiological data, and the seventh input is post-exercise physiological data. An output of the algorithm model Z is an exercise and health result. For example, an exercise and health result displayed on a user interface 1100 shown in FIG. 11A may be output.

Specifically, the algorithm model Z used to provide the exercise and health result may be a neural network model, and may be obtained through training based on a fourth training set. A confidence level may be higher than a specific confidence value, for example, 90%. The fourth training set includes a large quantity of training samples. One of these training samples may include an input and an output sample corresponding to the input. The input may include the pre-exercise physiological data, the in-exercise physiological data, and the post-exercise physiological data. The output sample may be the exercise and health result. The exercise and health result may usually be an exercise and health result that may be recognized by the medical field when a person performs the exercise and physiological data such as blood pressure, a heart rate, an electrocardiogram, and blood oxygen of the person resembles the pre-exercise physiological data, the in-exercise physiological data, and the post-exercise physiological data in the training sample. Therefore, the exercise and health result is usually trustworthy and can be used to train a neural network.

The exercise and health result may include a blood pressure evaluation result and cardiovascular risk prediction. It is easy to understand that the cardiovascular risk prediction may be expressed in a form of score and/or risk level (for example, a low risk, a medium risk, or a high risk).

### Display of exercise and health result

The exercise and health result may be presented in various forms. This is not limited in this embodiment of this application.

The electronic device 100 may display a brief report used to describe the exercise and health result, and send the exercise and health result to the electronic device 200. The electronic device 200 may display a detailed report used to describe the exercise and health result.

For example, after obtaining the exercise and health result of the user, the electronic device 100 may display a user interface 1100 shown in FIG. 11A, and provide blood pressure response evaluation and cardiovascular risk prediction on the user interface. The blood pressure response evaluation indicates a blood pressure level evaluation result of the user in an entire exercise process. For example, "slow blood pressure recovery" may indicate that the blood pressure level of the user recovers slowly. The cardiovascular risk prediction may indicate an exercise-related cardiovascular risk evaluation result of the user. For example, "medium risk" may indicate that the cardiovascular risk evaluation result of the user is in a non-good state.

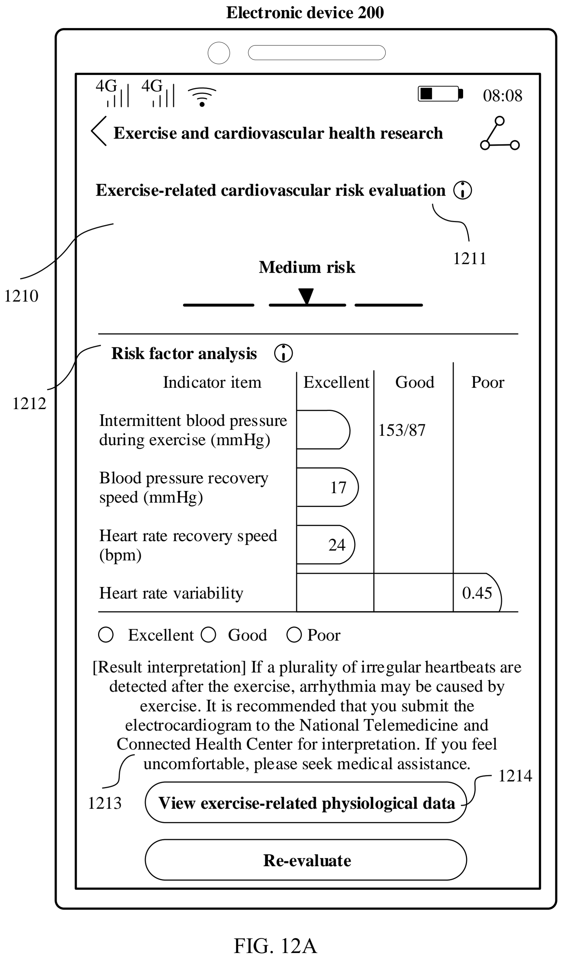

For example, after receiving the exercise and health result from the electronic device 100, the electronic device 200 may display a user interface 1210 shown in FIG. 12A, and provide indication information 1211, indication information 1212, and indication information 1213 on the user interface. The indication information 1211 may indicate an overall exercise and health risk of the user. For example, "medium risk" may indicate that the exercise and health result of the user is in a non-good state. The indication information 1212 may separately indicate impact of each risk factor on a health status of the user during the exercise. For example, "intermittent blood pressure during exercise", "blood pressure recovery speed", "heart rate recovery speed", and "heart rate variability" may respectively indicate a blood pressure status of the user when the user pauses the exercise during the exercise, a post-exercise blood pressure recovery status, a post-exercise heart rate recovery status, and a variation in a time interval between heartbeats. The indication information 1213 may be an interpretation of the indication information 1212, to help the user understand the indication information 1212.

It can be learned that the user interface 1210 shown in FIG. 12A focuses on presenting the exercise and health result of the user in terms of a risk level, a risk factor, and the like. The risk factor may be determined based on the in-exercise physiological data and the post-exercise physiological data, and the risk level may be determined based on a risk level of a risk factor evaluated based on the in-exercise physiological data and a risk level of a risk factor evaluated based on the post-exercise physiological data.

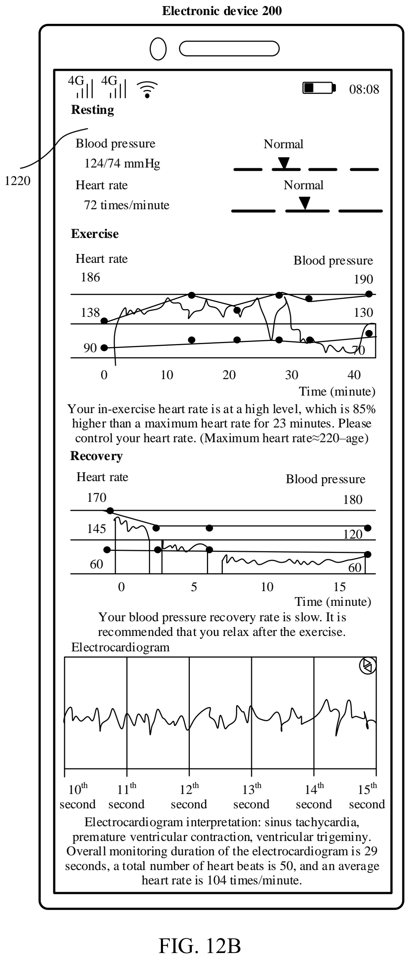

For example, after receiving the exercise and health result from the electronic device 100, the electronic device 200 may display a user interface 1220 shown in FIG. 12B, and provide, on the user interface, a physical health status like blood pressure and a heart rate during resting (before the exercise), a physical health status like blood pressure and a heart rate during the exercise, and a physical health status like blood pressure and a heart rate during the exercise recovery (after the exercise).

It can be learned that the user interface 1220 shown in FIG. 12B focuses on presenting the exercise and health results of the user at different stages.

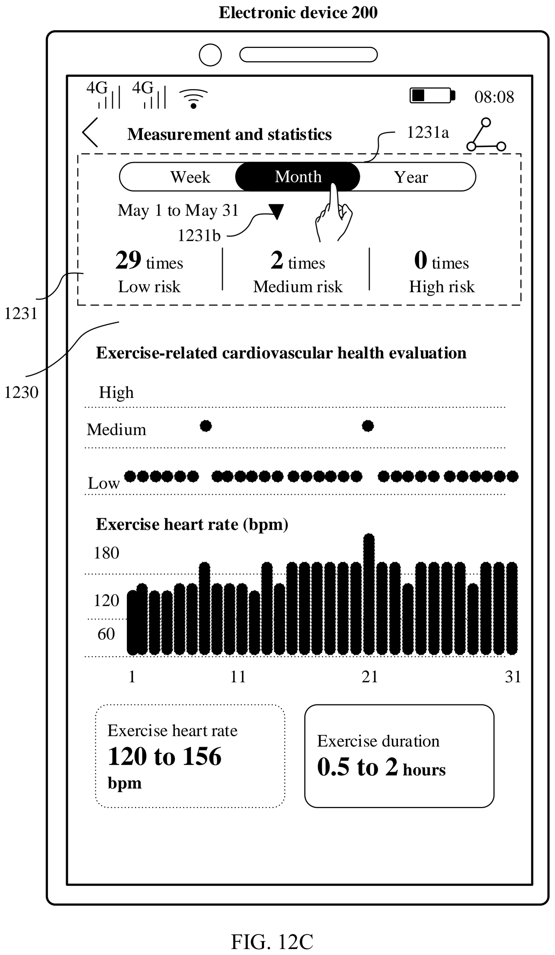

For example, after receiving the exercise and health result from the electronic device 100, the electronic device 200 may display a user interface 1230 shown in FIG. 12C, and display, on the user interface, statistics of exercise and health results of the user in a long period of time by using a graph, a table, a text, or the like, for example, exercise and health results from May 1 to May 31, where 29 exercises have low risks and 2 exercises have medium risks, and heart rate distribution of each exercise.

It can be learned that the user interface 1230 shown in FIG. 12C focuses on presenting the exercise and health result of the user in terms of measurement and statistics. Specifically, the user interface 1230 may display exercise and health results of a plurality of exercises performed by the user. The exercise and health results of the plurality of exercises may include one or more of the following: quantities of occurrences of different risk levels in the plurality of exercises, a risk level of each exercise, and physiological data collected during or after each exercise.

The following describes in detail how the electronic device 100 identifies an exercise stage of the user based on the collected exercise data.

### Exercise stage identification

The pre-exercise stage, the in-exercise stage, and the post-exercise stage can be identified in the following manners.

Manner 1: Identify each exercise stage based on collected exercise data.

Specifically, when the user is in the non-exercise state, and the electronic device 100 detects that duration of one or more of following data exceeds first duration (for example, 30 seconds): data that is collected by using the acceleration sensor and that indicates that an exercise speed of the user changes from a small value (for example, 0.1 meters/second) to a large value (for example, 5 meters/second), and data that is collected by the positioning sensor and that indicates that a location change of the user changes from a small value (for example, 10 meters/minute) to a large value (for example, 100 meters/minute), the electronic device 100 may determine that the user starts to exercise, and determine a change moment of the exercise data as a start moment of the exercise.

Specifically, when the user starts to exercise, and the electronic device 100 detects that duration of one or more of following data exceeds second duration (for example, 30 minutes): data that is collected by using the acceleration sensor and that indicates that an exercise speed of the user changes from a large value (for example, 5 meters/second) to a small value (for example, 0.1 meters/second), and data that is collected by the positioning sensor and that indicates that a location change of the user changes from a large value (for example, 100 meters/minute) to a small value (for example, 10 meters/minute), the electronic device 100 may determine that the user ends the exercise, and determine a change moment of the exercise data as an end moment of the exercise.

It is easy to understand that, on this basis, a period before the start moment of the exercise may be determined as pre-exercise, a period within preset duration (for example, within 20 minutes) after the end moment of the exercise may be determined as post-exercise, and a period between the start moment and the end moment may be determined as in-exercise.

### Manner 2: Identify each exercise stage based on a user input.

For example, when the user taps the icon 822 on the user interface 820 shown in FIG. 8B or an icon 831 on a user interface 830 shown in FIG. 8C, the electronic device 100 may determine that the user starts to exercise, and a moment at which the user taps the icon 822 or the icon 831 may be determined as a start moment of the exercise. When the user selects the stop control 911, the electronic device 100 may determine that the user ends the exercise, and a moment at which the user taps the stop control 911 may be determined as an end moment of the exercise. On this basis, a period before the start moment may be determined as pre-exercise, a period within preset duration (for example, within 20 minutes) after the end moment may be determined as post-exercise, and a period between the start moment and the end moment may be determined as in-exercise.

Based on the overall method procedure described in FIG. 5, the following describes in detail a series of graphical user interfaces provided in embodiments of this application. The graphical user interface is essentially a human-computer interaction method, and is an interface control method of the electronic device 100.

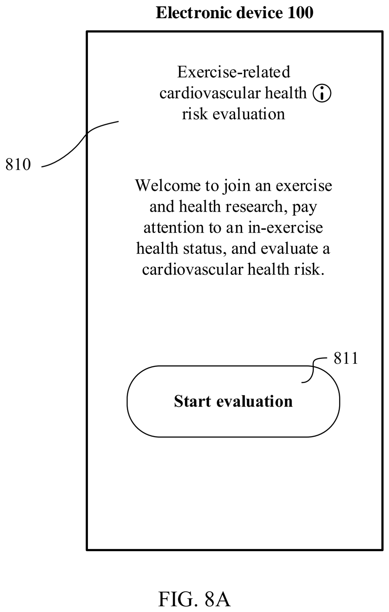

**FIG. 8A to FIG. 8G** **show examples of a series of user interfaces used by a user to measure pre-exercise physiological data.** **FIG. 9A to FIG. 9D** **show examples of a series of user interfaces used by a user to measure in-exercise physiological data.** **FIG. 10A to FIG. 10J** **show examples of a series of user interfaces used by a user to measure post-exercise physiological data.**

### Before exercise

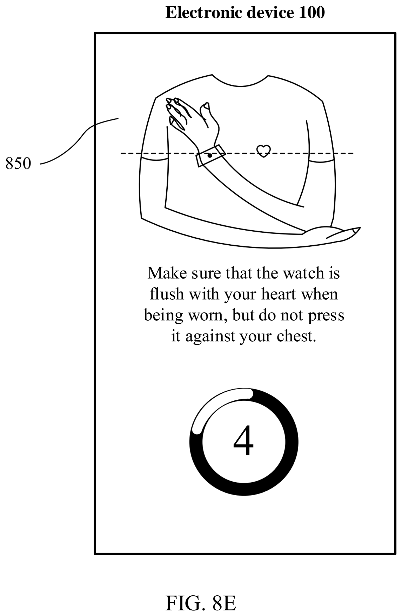

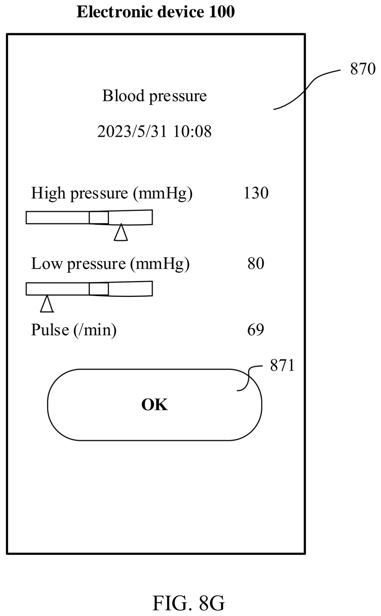

Before the exercise of the user, the electronic device 100 may display user interfaces shown in FIG. 8A to FIG. 8G. A first option may be displayed on the user interface, for example, the option 841 on the user interface 840 shown in FIG. 8D. When detecting that the user taps the first option, the electronic device 100 may start to collect the pre-exercise physiological data. For example, when detecting that the user taps a "Start measurement" option, the electronic device 100 may start to collect pre-exercise blood pressure data. In addition, the electronic device 100 may further display a related measurement interface, for example, the user interface 850 shown in FIG. 8E, the user interface 860 shown in FIG. 8F, and a user interface 870 shown in FIG. 8G. The foregoing interfaces are described below.

Further, before the pre-exercise physiological data starts to be collected, once it is detected, by using an exercise sensor like a gyroscope sensor or an acceleration sensor, that the user exercises, the electronic device 100 may display an "automatic identification of an exercise mode" user interface 830. This interface is described below. Before starting to collect the pre-exercise physiological data, the electronic device 100 may also display an "actively entering an exercise mode" user interface 820. This interface is described below.

It is easy to understand that when detecting that the user taps the first option, the electronic device 100 is not limited to starting to collect the blood pressure data, and may further start to collect pre-exercise physiological data like a heart rate, an electrocardiogram, and blood oxygen. This is not limited in this embodiment of this application.

As shown in FIG. 8A to FIG. 8G, in some embodiments, the electronic device 100 may automatically identify an exercise mode of the user by using the exercise sensor. In some other embodiments, the electronic device 100 may detect an input operation of the user. For example, the user may fill in a pre-exercise questionnaire (including an age, an exercise habit, a personal health status, a family medical history, medication information, and the like) when performing exercise and health detection by using the electronic device 100 for a first time, and the electronic device 100 may store basic user information. After detecting the input operation of the user, the electronic device 100 may add exercise-related data to research, for example, exercise and blood pressure research. After adding the exercise-related data to the research, the electronic device 100 may prompt the user to start an exercise and health detection procedure (for example, an exercise-related cardiovascular risk evaluation procedure). The user may choose to start the exercise and health detection procedure. After the exercise and health detection procedure is started, the user may choose to actively enter an exercise mode.

After detecting the exercise mode of the user, the electronic device 100 may prompt the user to start to collect the pre-exercise physiological data (for example, blood pressure). When starting to collect the pre-exercise physiological data, the electronic device 100 may display a guide interface (the user interface 850 shown in FIG. 8E and the user interface 860 shown in FIG. 8F) for collecting the physiological data, to improve accuracy of collecting the physiological data of the user. For example, the user is prompted with "Make sure that the watch is flush with your heart when being worn, but do not press it against your chest" and "Please stay still during blood pressure measurement".

For example, as shown in FIG. 8C, the "automatic identification of an exercise mode" user interface 830 may include one or more exercise mode icons (for example, an indoor walking icon, an outdoor walking icon, an indoor running icon, an outdoor running icon 831, an elliptical machine icon, and a rowing machine icon), and one or more options (for example, an "Ignore" option, an "Ignore today" option, and a "Disable automatic identification" option). The exercise mode icon may be used to trigger the electronic device 100 to determine that the user starts to exercise. For example, the outdoor running icon 831 may be used to trigger the electronic device 100 to determine that the user starts to enter an outdoor running exercise mode.

In some embodiments, the electronic device 100 may receive an input operation (for example, a tap) performed by the user on the exercise mode icon (for example, the outdoor running icon 831). In response to the input operation, the electronic device 100 may display the user interface 840 shown in FIG. 8D. In the foregoing embodiment, a physiological status of the user at the beginning of the exercise is similar to a resting state. Therefore, the physiological data collected in this case is similar to the physiological data of the user in the resting state, and may be considered as the pre-exercise physiological data. In some other embodiments, the user may not perform inputting on the exercise mode icon. After preset duration (for example, 30 seconds), the electronic device 100 may determine that the user has started to enter the exercise mode, and then may automatically enter the user interface 840 shown in FIG. 8D.

For example, as shown in FIG. 8A, the electronic device 100 may display a "start evaluation prompt" user interface 810. Optionally, the user interface 810 may include related information used to prompt the user to start the exercise-related cardiovascular risk evaluation procedure, for example, "Welcome to join an exercise and health research, pay attention to an in-exercise health status, and evaluate a cardiovascular health risk". It can be learned from the prompt information that the electronic device 100 prompts the user that data of this exercise may be added to the research (for example, the exercise and health research). A purpose of starting the exercise-related cardiovascular risk evaluation procedure is to prompt the user to pay attention to an exercise and health result. In some examples, the prompt information may not be conveyed to the user in an interface displaying manner, but may be conveyed to the user in another manner, for example, may be conveyed to the user in a voice broadcasting manner. The user interface 810 may further include an option 811. The option 811 may be used to trigger initiation of the exercise-related cardiovascular risk evaluation procedure.

The electronic device 100 may receive an input operation (for example, a tap) performed by the user on the option 811. In response to the input operation, the electronic device 100 may display the "actively entering an exercise mode" user interface 820 shown in FIG. 8B.

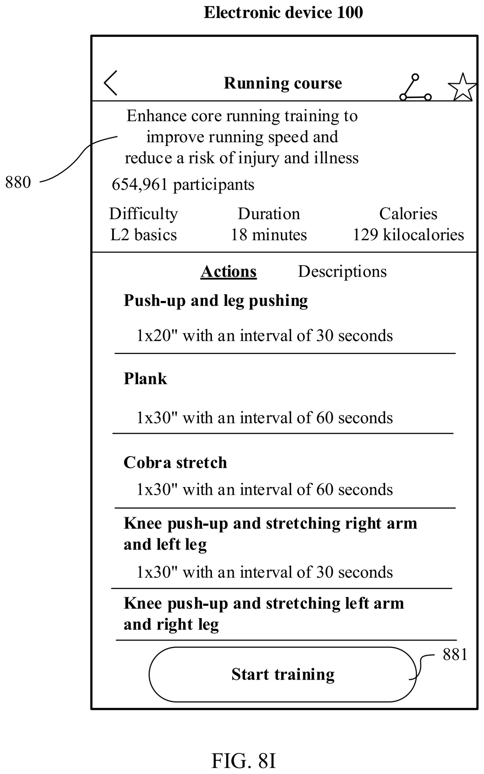

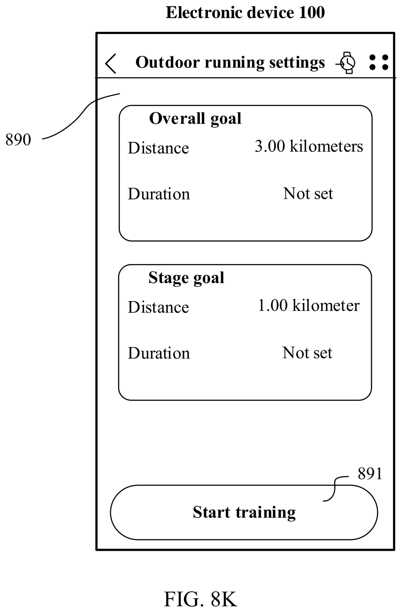

For example, as shown in FIG. 8B, the electronic device 100 may display the "actively entering an exercise mode" user interface 820. The user interface 820 may include one or more course icons (for example, a running course icon), an access control (for example, a control 821) corresponding to the course icon, one or more exercise mode icons (for example, an outdoor running icon 822), and a setting control (for example, a control 823) corresponding to the exercise mode icon. The access control corresponding to the course icon may be used to trigger access to an exercise course interface (for example, a user interface 880 shown in FIG. 8I). The exercise mode icon may be used to trigger the electronic device 100 to determine that the user starts to exercise. For example, the outdoor running icon 822 may be used to trigger the electronic device 100 to determine that the user starts to enter an outdoor running exercise mode. The setting control corresponding to the exercise mode icon may be used to trigger entering an exercise mode setting interface (for example, a user interface 890 shown in FIG. 8K).

The electronic device 100 may receive an input operation (for example, a tap) performed by the user on the exercise mode icon. In response to the input operation, the electronic device 100 may display the user interface 840 shown in FIG. 8D.

In some embodiments, the electronic device 100 may establish a connection to an electronic device other than the electronic device 100, for example, establish a connection to the electronic device 200. The electronic device 100 may read an exercise course played by the electronic device 200. In some other embodiments, the electronic device 100 may further read an exercise course played by the electronic device 100. When actively entering the exercise mode, the user may choose to enter the exercise course read by the electronic device 100.

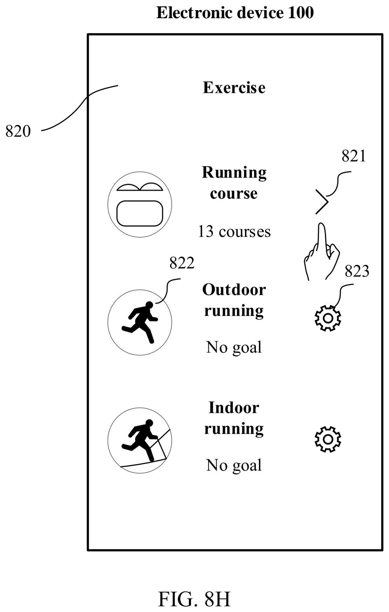

For example, as shown in FIG. 8H, the electronic device 100 may receive an input operation (for example, a tap) performed by the user on the control 821. In response to the input operation, the electronic device 100 may display the user interface 880 shown in FIG. 8I.

The electronic device 100 may remind the user to measure the in-exercise physiological data, such as blood pressure, an electrocardiogram, and blood oxygen during a rest interval of a course (for example, "rest for 60 seconds" on the user interface 880 shown in FIG. 8I) or after the course ends (for example, "duration of 18 minutes" on the user interface 880 shown in FIG. 8I).

For example, as shown in FIG. 8I, the electronic device 100 may display a "running course" user interface 880, and the user interface 880 may include an option 881. The option 881 may be used to trigger the electronic device 100 to determine that the user starts to enter a running course, that is, enter a running exercise mode.

The electronic device 100 may receive an input operation (for example, a tap) performed by the user on the option 881. In response to the input operation, the electronic device 100 may display the user interface 840 shown in FIG. 8D.

In some embodiments, when actively entering the exercise mode, the user may select to customize an overall goal and a stage goal of the exercise mode.

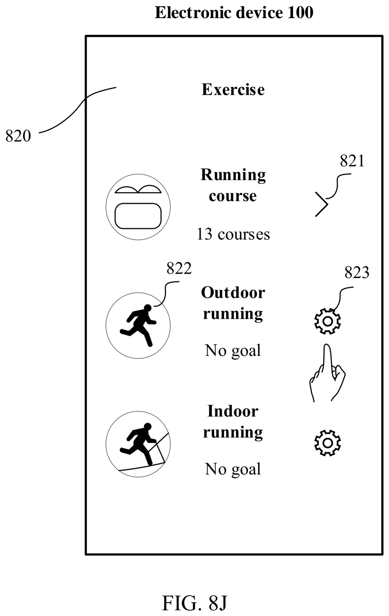

For example, as shown in FIG. 8J, the electronic device 100 may receive an input operation (for example, a tap) performed by the user on the control 823. In response to the input operation, the electronic device 100 may display the user interface 890 shown in FIG. 8K.

The electronic device 100 may remind the user to measure the in-exercise physiological data, such as blood pressure, a heart rate, and blood oxygen, after the user achieves the preset phased goal (for example, one or more conditions such as an outdoor running distance of 1 kilometer and outdoor running duration of 10 minutes) or after the user achieves the preset overall goal (for example, one or more conditions such as an overall outdoor running distance of 3 kilometers and overall outdoor running duration of 30 minutes). It may be understood that setting of the exercise goal shown in FIG. 8K is merely an example, and is not limited thereto. Content (such as a distance and duration on the user interface 890 shown in FIG. 8K) of the exercise goal (including the overall goal and the stage goal) may be related to the exercise mode of the user. Content of the overall goal and the stage goal may be usually consistent.

For example, as shown in FIG. 8K, the electronic device 100 may display the user interface 890, and the user interface 890 may include an option 891. The option 891 may be used to trigger the electronic device 100 to determine that the user starts to enter the outdoor running exercise mode.

The electronic device 100 may receive an input operation (for example, a tap) performed by the user on the option 891. In response to the input operation, the electronic device 100 may display the user interface 840 shown in FIG. 8D.

For example, as shown in FIG. 8D, the electronic device 100 may display the user interface 840. Optionally, the user interface 840 may include related information used to prompt the user to collect the pre-exercise physiological data, for example, "Please measure pre-exercise blood pressure. Please stay still and start measurement". It can be learned from the prompt information that the electronic device 100 prompts the user that blood pressure can be measured once before the exercise, and prompts the user that an action that needs to be performed is to stay still. In some examples, the prompt information may not be conveyed to the user in an interface displaying manner, but may be conveyed to the user in another manner, for example, may be conveyed to the user in a voice broadcasting manner. The user interface 840 may further include the option 841. The option 841 may be used to trigger collection of the pre-exercise physiological data such as blood pressure and a heart rate.

Blood pressure data collection is used as an example. The electronic device 100 may receive an input operation (for example, a tap) performed by the user on the option 841. In response to the input operation, the electronic device 100 may start to collect pre-exercise blood pressure data, and display the user interface 850 shown in FIG. 8E.

For example, as shown in FIG. 8E, the electronic device 100 may display the user interface 850. Optionally, the user interface 850 may include related information used to prompt the user to perform a blood pressure measurement preparation action, for example, an action diagram and prompt information (for example, "Make sure that the watch is flush with your heart when being worn, but do not press it against your chest"). It can be learned from the foregoing prompt information that, when prompting the user to measure blood pressure, the electronic device 100 needs to note that a wearing position of the watch is flush with the heart, and the chest cannot be pressed, to improve accuracy of blood pressure data obtained through measurement. In some examples, the prompt information may not be conveyed to the user in an interface displaying manner, but may be conveyed to the user in another manner, for example, may be conveyed to the user in a voice broadcasting manner. The user interface 850 may further include a countdown control. The countdown control may be used to prompt the user to wait for preset duration, to trigger entering of the user interface 860 after the countdown ends. The preset duration may be 30 seconds, and a specific value of the preset duration is not limited.

For example, as shown in FIG. 8F, the electronic device 100 may display the user interface 860. Optionally, the user interface 860 may include related information that indicates blood pressure measurement data. The user interface 860 may include related prompt information (for example, "Please stay still during blood pressure measurement") used to prompt the user to perform an action in a blood pressure measurement process. It can be learned from the foregoing prompt information that an action that needs to be performed when the electronic device 100 prompts the user to measure blood pressure is to stay still, to improve accuracy of blood pressure data obtained through measurement. In some examples, the prompt information may not be conveyed to the user in an interface displaying manner, but may be conveyed to the user in another manner, for example, may be conveyed to the user in a voice broadcasting manner. The user interface 860 may further include a "Cancel measurement" option, and the option may be used to cancel an ongoing blood pressure measurement procedure.

In some embodiments, after the blood pressure measurement ends after the preset duration, the electronic device 100 may enter the user interface 870. The preset duration may be 2 minutes, and a specific value of the preset duration is not limited in this embodiment of this application.

For example, as shown in FIG. 8G, the electronic device 100 may display the user interface 870. The user interface 870 may include related information (for example, high pressure, low pressure, and a pulse on the user interface 870 shown in FIG. 8G) that indicates a blood pressure measurement result of the user. It is easy to understand that the indication information is merely an example, and is not limited thereto. Content of the blood pressure measurement result is not limited in this embodiment of this application. The user interface 870 may further include an option 871, and the option 871 may be used to trigger ending of the blood pressure measurement procedure.

The electronic device 100 may receive an input operation (for example, a tap) performed by the user on the option 871. In response to the input operation, the electronic device 100 may display the user interface 910 shown in FIG. 9A.

### During exercise

During the exercise of the user, the electronic device 100 may display user interfaces shown in FIG. 9A to FIG. 9D. As described above, the exercise mentioned in this embodiment of this application may include an interval at which the user pauses the exercise. In this interval, the electronic device 100 may better collect physiological data that requires the user to be in a still state, such as electrocardiogram data, blood pressure data, and blood oxygen data. A second option may be displayed on the user interface, for example, the option 921 and the option 922 on the user interface 920 shown in FIG. 9B. When detecting that the user taps the second option, the electronic device 100 may start to collect the in-exercise physiological data. For example, when detecting that the user taps a "blood pressure measurement" option, the electronic device 100 may start to collect in-exercise blood pressure data. For another example, when detecting that the user taps an "electrocardiogram collection" option, the electronic device 100 may start to collect in-exercise electrocardiogram data. In addition, the electronic device 100 may further display a related measurement interface. For example, for a measurement interface related to blood pressure measurement, refer to FIG. 8E to FIG. 8G. Details are not described herein again. For another example, for a measurement interface related to electrocardiogram collection, refer to FIG. 10H. Details are not described herein.

Specifically, the in-exercise physiological data may be collected at an interval at which the user pauses the exercise.

Specifically, the in-exercise physiological data may also be collected in one or more of the following scenarios: The electronic device 100 may remind, after reading a resting time period of an exercise course of the electronic device 100 or the electronic device 200, the user to measure the in-exercise physiological data; or the electronic device 100 may remind the user to measure the in-exercise physiological data after the user reaches a preset phased goal (for example, one or more conditions such as an outdoor running distance of 1 kilometer, and outdoor running duration of 10 minutes).

In some embodiments, the electronic device 100 may collect exercise data in a process in which the user exercises. Further, during the exercise, the electronic device 100 may input the in-exercise physiological data and the exercise data into the algorithm model Y. Once it is detected that one or more of the in-exercise physiological data such as blood pressure, a heart rate, an electrocardiogram, and blood oxygen is abnormal, that is, one or more of the in-exercise physiological data such as blood pressure, a heart rate, an electrocardiogram, and blood oxygen deviates from normal physiological data in the exercise mode selected by the user, the electronic device 100 may display the user interface 930 shown in FIG. 9C or the user interface 940 shown in FIG. 9D, where the user interface may include prompt information, and the prompt information indicates a health abnormality in a process in which the user performs the first exercise. The normal physiological data is a reference value that is of physiological data obtained when the user performs the first exercise and that is determined based on the pre-exercise physiological data. The prompt information may be generated based on the in-exercise physiological data, or based on the pre-exercise physiological data and the in-exercise physiological data. The health abnormality includes one or more of the following: an abnormally high blood pressure, an abnormally low blood pressure, a retarded reaction, and arrhythmia.

In some embodiments, the prompt information may indicate a risk existing when the user performs the selected exercise mode. Specifically, that one or more of the in-exercise physiological data such as blood pressure, a heart rate, an electrocardiogram, and blood oxygen is abnormal may indicate that one or more of the in-exercise physiological data such as blood pressure, a heart rate, an electrocardiogram, and blood oxygen in the exercise mode selected by the user deviates from normal physiological data. In other words, a risk level of performing the selected exercise mode by the user is high.

As shown in FIG. 9A to FIG. 9D, after the electronic device 100 completes collection of the pre-exercise physiological data such as blood pressure and a heart rate, the user may enter a period in which the exercise is performed. In this period, the user may pause the exercise. When detecting that the user is in a period (which may also be referred to as an exercise interval) in which the exercise is paused, the electronic device 100 may prompt the user to start to collect the in-exercise physiological data (for example, blood pressure and an electrocardiogram). When starting to collect the in-exercise physiological data, the electronic device 100 may display a related measurement interface. For example, for a measurement interface related to blood pressure measurement, refer to FIG. 8E to FIG. 8G. Details are not described herein again. For another example, for a measurement interface related to electrocardiogram collection, refer to FIG. 10H. Details are not described herein. The electronic device 100 may collect exercise data in a process in which the user exercises. During the exercise, the electronic device 100 may input the in-exercise physiological data and the exercise data into the algorithm model Y. Once it is detected that one or more of the in-exercise physiological data such as blood pressure, a heart rate, an electrocardiogram, and blood oxygen is abnormal, the electronic device 100 may trigger an abnormal event reminder.

For example, as shown in FIG. 9A, the electronic device 100 may display the user interface 910. The user interface 910 may include related information that indicates heart rate data and exercise data (for example, "pace", "distance", and "duration" on the user interface 910 shown in FIG. 9A) of the user in in-exercise. It is easy to understand that the indication information on the user interface 910 shown in FIG. 9A is merely an example, and is not limited thereto. Content of the exercise data in the indication information is related to the exercise mode of the user. The user interface 910 may further include a pause control 911 and a stop control 912. The pause control 911 may be used by the user to actively pause the exercise. The stop control 912 may be used by the user to actively end the exercise.

In some embodiments, the electronic device 100 may receive an input operation (for example, a tap) performed by the user on the pause control 911. In response to the input operation, the electronic device 100 may display the user interface 920 shown in FIG. 9B. In some other embodiments, the electronic device 100 may receive an input operation (for example, a tap) performed by the user on the stop control 912. In response to the input operation, the electronic device 100 may display the user interface 1010 shown in FIG. 10A.

For example, as shown in FIG. 9B, the electronic device 100 may display the user interface 920. Optionally, the user interface 920 may include related information used to prompt the user to collect the in-exercise physiological data, for example, "The exercise is paused, and blood pressure measurement or electrocardiogram collection may be performed once". It can be learned from the prompt information that the electronic device 100 prompts the user that blood pressure can be measured or an electrocardiogram can be collected in the period in which the exercise is paused. In some examples, the prompt information may not be conveyed to the user in an interface displaying manner, but may be conveyed to the user in another manner, for example, may be conveyed to the user in a voice broadcasting manner. The user interface 920 may further include the option 921, the option 922, and a "blood oxygen measurement" option (not shown in the figure). The option 921 may be used to trigger collection of intermittent blood pressure during exercise, and the intermittent blood pressure during exercise is relative to blood pressure that is continuously collected when the exercise of the user is not interrupted. The option 922 may be used to trigger collection of the in-exercise physiological data such as an electrocardiogram. The "blood oxygen measurement" option may be used to trigger collection of the in-exercise physiological data such as blood oxygen.

In some embodiments, the electronic device 100 may receive an input operation (for example, a tap) performed by the user on the option 921. In response to the input operation, the electronic device 100 may display the measurement interface related to blood pressure measurement, for example, FIG. 8E to FIG. 8G. Details are not described herein. In some other embodiments, the electronic device 100 may receive an input operation (for example, a tap) performed by the user on the option 922. In response to the input operation, the electronic device 100 may display the measurement interface related to electrocardiogram collection, for example, FIG. 10H. Details are not described herein.

In some embodiments, after the user completes the blood pressure measurement procedure, the electrocardiogram collection procedure, or the blood oxygen measurement procedure, if it is detected that the blood pressure is abnormal, the electronic device 100 may display the user interface 930 shown in FIG. 9C; or if it is detected that the electrocardiogram is abnormal, the electronic device 100 may display the user interface 940 shown in FIG. 9D; otherwise, the electronic device 100 may display the user interface 910 shown in FIG. 9A.

For example, as shown in FIG. 9C, the electronic device 100 may display the user interface 930. The user interface 930 may include related information used to prompt that the blood pressure is abnormal, for example, "Currently, it is detected that your blood pressure is abnormally high/low. Because the blood pressure is abnormally high/low, XXX cannot be performed. Please take a break and re-measure your blood pressure. If you feel uncomfortable, please seek medical assistance". The blood pressure health abnormality in the prompt information may be, for example, abnormally high/low blood pressure. The prompt information may further include an in-exercise exercise suggestion for the user. The in-exercise exercise suggestion may be, for example, continuing exercise/reducing exercise intensity/increasing exercise intensity/stopping exercise/continuing exercise after resting for XX minutes/continuing exercise after resting for XX minutes if no abnormality is detected. In some examples, the prompt information may not be conveyed to the user in an interface displaying manner, but may be conveyed to the user in another manner, for example, may be conveyed to the user in a voice broadcasting manner. The user interface 930 may further include the option 931. The option 931 may be used to trigger collection of the in-exercise physiological data (for example, blood pressure).

For example, as shown in FIG. 9D, the electronic device 100 may display the user interface 940. The user interface 940 may include related information used to prompt an electrocardiogram abnormality, for example, "Your arrhythmia is detected. Arrhythmia can affect accuracy of blood pressure measurement. If you feel uncomfortable, please seek medical assistance". The electrocardiogram health abnormality in the prompt information may be, for example, arrhythmia. The prompt information may further include an in-exercise exercise suggestion for the user. The in-exercise exercise suggestion may be, for example, continuing exercise/reducing exercise intensity/increasing exercise intensity/stopping exercise/continuing exercise after resting for XX minutes/continuing exercise after resting for XX minutes if no abnormality is detected. In some examples, the prompt information may not be conveyed to the user in an interface displaying manner, but may be conveyed to the user in another manner, for example, may be conveyed to the user in a voice broadcasting manner. The user interface 940 may further include the option 941. The option 941 may be used to trigger collection of the in-exercise physiological data (for example, an electrocardiogram).

In some embodiments, the electronic device 100 may receive an input operation (for example, a tap) performed by the user on the option 931. In response to the input operation, the electronic device 100 may display the measurement interface related to blood pressure measurement, for example, FIG. 8E to FIG. 8G. Details are not described herein. Alternatively, the electronic device 100 may receive an input operation (for example, a tap) performed by the user on the option 941. In response to the input operation, the electronic device 100 may display the measurement interface related to electrocardiogram collection, for example, FIG. 10H. Details are not described herein.

### After exercise

After the exercise of the user, the electronic device 100 may display user interfaces shown in FIG. 10A to FIG. 10J. Post-exercise may be a period within preset duration (for example, within 20 minutes) after the exercise ends. After the exercise of the user ends, physiological data such as blood pressure and a heart rate usually gradually returns to a resting level within the preset duration. Therefore, post-exercise mentioned in this embodiment of this application is a post-exercise recovery period.

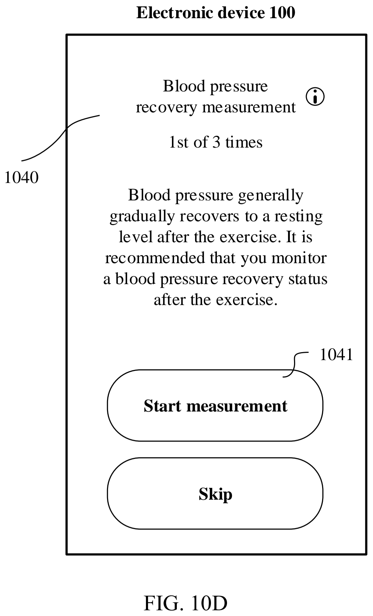

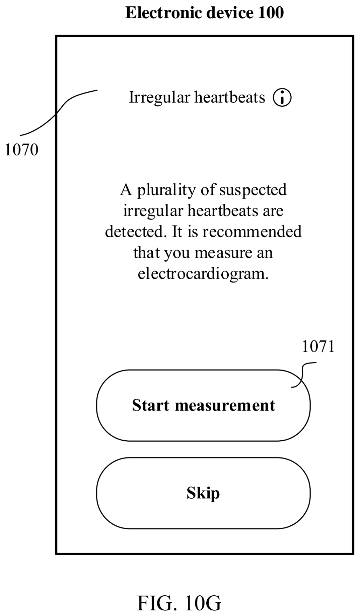

A third option may be displayed on the user interface, for example, the option 1011 on the user interface 1010 shown in FIG. 10A, an option 1041 on a user interface 1040 shown in FIG. 10D, and an option 1071 on a user interface 1070 shown in FIG. 10G. When detecting that the user taps the third option, the electronic device 100 may start to collect the post-exercise physiological data. For example, when detecting that the user taps a "Start measurement" option on the user interface 1010, the electronic device 100 may start to collect post-exercise blood pressure data. For another example, when detecting that the user taps a "Start measurement" option on the user interface 1040, the electronic device 100 may start to collect post-exercise blood pressure recovery data. For another example, when detecting that the user taps a "Start measurement" option on the user interface 1070, the electronic device 100 may start to collect post-exercise electrocardiogram data. In addition, the electronic device 100 may further display a related measurement interface. For example, for a measurement interface related to blood pressure measurement, refer to FIG. 8E to FIG. 8G. Details are not described herein again. For another example, for a measurement interface related to electrocardiogram collection, refer to FIG. 10H. The interface is described below.

Specifically, the post-exercise physiological data may be collected within the preset duration (for example, within 20 minutes) after the exercise of the user ends.

Specifically, the post-exercise physiological data may also be collected in one or more of the following scenarios: The electronic device 100 may remind, after reading an ending time period of an exercise course of the electronic device 100 or the electronic device 200, the user to measure the post-exercise physiological data; or the electronic device 100 may remind the user to measure the post-exercise physiological data after the user reaches a preset overall goal (for example, one or more conditions such as an overall outdoor running distance of 3 kilometer, and overall outdoor running duration of 30 minutes).

As shown in FIG. 10B, in some embodiments, post-exercise heart rate data may be automatically collected, and the user does not need to tap a measurement option.

In some embodiments, the post-exercise blood pressure recovery data may be collected a plurality of times, to improve accuracy of the collected post-exercise blood pressure recovery data.

Further, because the electrocardiogram data can more accurately reflect a cardiopulmonary function status of the user than the heart rate data, after the exercise, when an abnormality of the heart rate data is detected a plurality of times, the electronic device 100 may display the user interface 1070, to prompt the user that the electrocardiogram can be measured. This interface is described below.

As shown in FIG. 10A to FIG. 10J, after the electronic device 100 completes collection of the in-exercise physiological data such as blood pressure, a heart rate, an electrocardiogram, and blood oxygen, the user may end the exercise. After detecting that the user ends the exercise, the electronic device 100 may prompt the user to start to collect the post-exercise physiological data such as blood pressure and a heart rate. When starting to collect the post-exercise physiological data, the electronic device 100 may display a related measurement interface. For example, for a measurement interface related to blood pressure measurement, refer to FIG. 8E to FIG. 8G. Details are not described herein again. For another example, for a measurement interface related to heart rate measurement, refer to FIG. 10C. When detecting a plurality of suspected irregular heartbeats after the exercise, the electronic device 100 may prompt the user to measure an ECG (electrocardiogram). When starting to collect the electrocardiogram, the electronic device 100 may display a related measurement interface, as shown in FIG. 10H. After the electronic device 100 completes collection of the post-exercise physiological data such as blood pressure, a heart rate, an electrocardiogram, and blood oxygen, the electronic device 100 may input the collected pre-exercise physiological data, the collected in-exercise physiological data, and the collected post-exercise physiological data of the user into the algorithm model Z, the algorithm model Z may output an exercise and health result of the user.

For example, as shown in FIG. 10A, the electronic device 100 may display the user interface 1010. Optionally, the user interface 1010 may include related information used to prompt the user to collect the post-exercise physiological data, for example, "The exercise has ended. Please measure blood pressure immediately". It can be learned from the prompt information that the electronic device 100 prompts the user that blood pressure may be measured once immediately after the exercise ends. In some examples, the prompt information may not be conveyed to the user in an interface displaying manner, but may be conveyed to the user in another manner, for example, may be conveyed to the user in a voice broadcasting manner. The user interface 1010 may include the option 1011. The option 1011 may be used to trigger collection of the post-exercise physiological data such as blood pressure, so that the electronic device 100 obtains a maximum value of the post-exercise physiological data such as blood pressure in the entire exercise process.

In some embodiments, the electronic device 100 may receive an input operation (for example, a tap) performed by the user on the option 1011. In response to the input operation, the electronic device 100 may start to collect post-exercise blood pressure data, and display the user interface 850 shown in FIG. 8E. After the user completes collection of the post-exercise blood pressure data, the electronic device 100 may display the user interface 1020 shown in FIG. 10B.

For example, as shown in FIG. 10B, the electronic device 100 may display the user interface 1020. Optionally, the user interface 1020 may include related information used to prompt the user to perform heart rate recovery measurement, for example, "A heart rate generally gradually recovers to a resting level after the exercise. It is recommended that you monitor a heart rate recovery status after the exercise". It can be learned from the prompt information that the electronic device 100 prompts the user that heart rate recovery can be measured after the exercise, and a quantity of times of measuring heart rate recovery is not limited. In some examples, the prompt information may not be conveyed to the user in an interface displaying manner, but may be conveyed to the user in another manner, for example, may be conveyed to the user in a voice broadcasting manner. The user interface 1020 may further include a countdown control. The countdown control may be used to prompt the user to wait for preset duration, to trigger entering of the user interface 1030 after the countdown ends. The preset duration needs to be less than or equal to the preset duration after the exercise of the user ends, and the preset duration may be 30 seconds. A specific value of the preset duration is not limited in this embodiment of this application.

For example, as shown in FIG. 10C, the electronic device 100 may display the user interface 1030. The user interface 1030 may include related information that indicates heart rate recovery measurement data. The user interface 1030 may further include action prompt information (for example, "Please stay still for 2 minutes") used to prompt the user to perform heart rate recovery measurement. It can be learned from the foregoing prompt information that an action that needs to be performed when the electronic device 100 prompts the user to measure heart rate recovery is to stay still, and duration for which the electronic device 100 prompts the user to stay still may be, for example, 2 minutes. It may be understood that, that the duration for measuring heart rate recovery is 2 minutes is merely an example. This is not limited in this embodiment of this application. In some examples, the prompt information may not be conveyed to the user in an interface displaying manner, but may be conveyed to the user in another manner, for example, may be conveyed to the user in a voice broadcasting manner.

For example, as shown in FIG. 10D, the electronic device 100 may display the user interface 1040. Optionally, the user interface 1040 may include related information used to prompt the user to perform blood pressure recovery measurement for the first time after the exercise, for example, "Blood pressure generally gradually recovers to a resting level after the exercise. It is recommended that you monitor a blood pressure recovery status after the exercise". It can be learned from the prompt information that the electronic device 100 prompts the user that blood pressure recovery can be measured after the exercise. In some examples, the prompt information may not be conveyed to the user in an interface displaying manner, but may be conveyed to the user in another manner, for example, may be conveyed to the user in a voice broadcasting manner. The user interface 1040 may include the option 1041. The option 1041 may be used to trigger collection of post-exercise blood pressure recovery data.

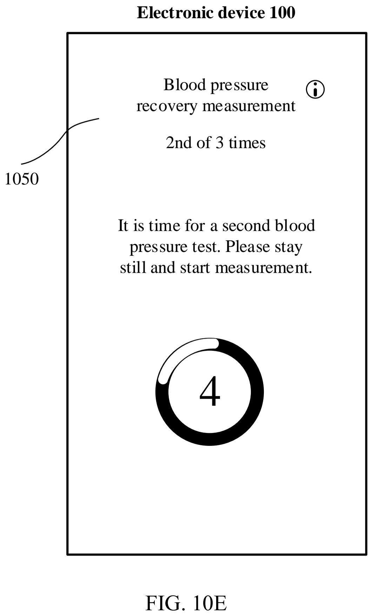

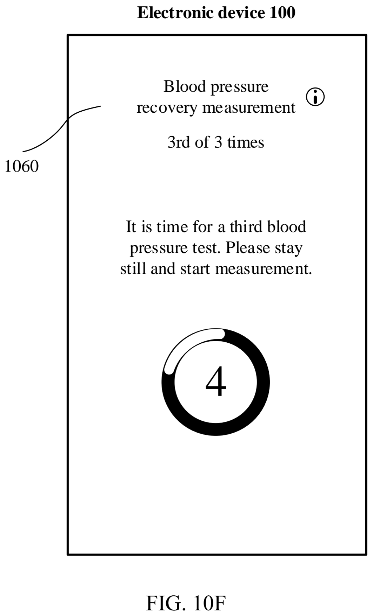

In some embodiments, the electronic device 100 may receive an input operation (for example, a tap) performed by the user on the option 1041. In response to the input operation, the electronic device 100 may start to collect the post-exercise blood pressure recovery data for the first time, and display the user interface 850 shown in FIG. 8E. After the user completes collection of the post-exercise blood pressure recovery data for the first time, the electronic device 100 may wait until a countdown of preset duration (for example, 30 seconds) on a user interface 1050 shown in FIG. 10E ends, and then continue to start to collect the post-exercise blood pressure recovery data, for example, the post-exercise blood pressure recovery data for the second time. After the user completes collection of the post-exercise blood pressure recovery data for the second time, the electronic device 100 may wait until a countdown of preset duration (for example, 30 seconds) on a user interface 1060 shown in FIG. 10F ends, and then continue to start to collect the post-exercise blood pressure recovery data, for example, the post-exercise blood pressure recovery data for the third time.

In a gap of measuring the post-exercise blood pressure recovery, the electronic device 100 may continuously collect the post-exercise heart rate recovery of the user. The gap may be preset duration (for example, 30 seconds) from a start of the countdown to an end of the countdown, for example, duration from an end of the first time of measurement of the post-exercise blood pressure recovery to a start of the second time of measurement of the post-exercise blood pressure recovery. Continuous collection may include uninterrupted collection and a plurality of collections at a specific time interval (for example, 1 minutes).

In some embodiments, a post-exercise heart rate recovery speed of the user may be determined based on one or more of the following: heart rate data at an end moment of the exercise, heart rate data collected by using the barometric pressure sensor, the micro pump, and the airbag, heart rate data collected by using the ECG sensor, and heart rate data collected by using the PPG sensor. A heart rate at the end moment of the exercise may be considered as a maximum heart rate in the entire exercise process.

After completing collection of the post-exercise blood pressure recovery data, the electronic device 100 may prompt the user to fill in a post-exercise questionnaire. The post-exercise questionnaire may include information such as a degree of fatigue and an abnormal symptom of the user after the user ends the exercise.

For example, as shown in FIG. 10G, the electronic device 100 may display the user interface 1070. The user interface 1070 may include related information used to prompt a plurality of suspected irregular heartbeats and suggest ECG measurement, for example, "A plurality of suspected irregular heartbeats are detected. It is recommended that you measure an electrocardiogram". In some examples, the prompt information may not be conveyed to the user in an interface displaying manner, but may be conveyed to the user in another manner, for example, may be conveyed to the user in a voice broadcasting manner. The user interface 1070 may include the option 1071. The option 1071 may be used to trigger collection of post-exercise ECG data.

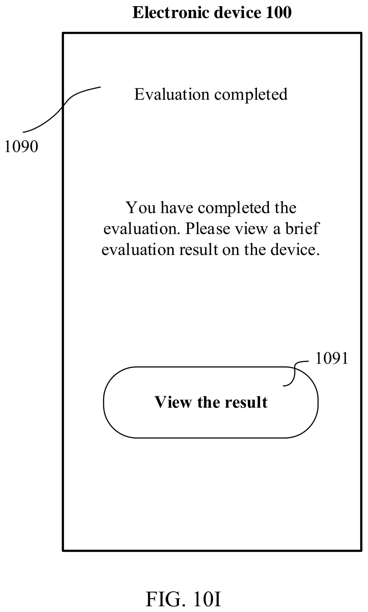

In some embodiments, the electronic device 100 may receive an input operation (for example, a tap) performed by the user on the option 1071. In response to the input operation, the electronic device 100 may start to collect post-exercise electrocardiogram data, and display the user interface 1080 shown in FIG. 10H. After the user completes collection of the post-exercise electrocardiogram data, the electronic device 100 may display a user interface 1090 shown in FIG. 10I.

For example, as shown in FIG. 10H, the electronic device 100 may display a user interface 1080. Optionally, the user interface 1080 may include related information used to prompt the user with an electrocardiogram collection action, for example, prompt information "Wear with a left hand", "Please touch and hold a side electrode", and a corresponding diagram of an action. It can be learned from the foregoing prompt information that the electronic device 100 may prompt the user that an action that needs to be performed during ECG measurement is to lightly touch the side electrode of the electronic device 100 and hold for preset duration, and prompt the user that the electronic device 100 needs to be worn with the left hand, to improve accuracy of the collected electrocardiogram data. The preset duration may be 30 seconds, and a specific value of the preset duration is not limited in this embodiment of this application. In some examples, the prompt information may not be conveyed to the user in an interface displaying manner, but may be conveyed to the user in another manner, for example, may be conveyed to the user in a voice broadcasting manner.

For example, as shown in FIG. 10I, the electronic device 100 may display the user interface 1090. The user interface 1090 may include related information used to prompt the user to complete evaluation and view a result. The prompt information may be, for example, "You have completed the evaluation. Please view a brief evaluation result on the device". In some examples, the prompt information may not be conveyed to the user in an interface displaying manner, but may be conveyed to the user in another manner, for example, may be conveyed to the user in a voice broadcasting manner. The user interface 1090 may include the option 1091. The option 1091 may be used to trigger jumping to a display interface of the exercise and health result.

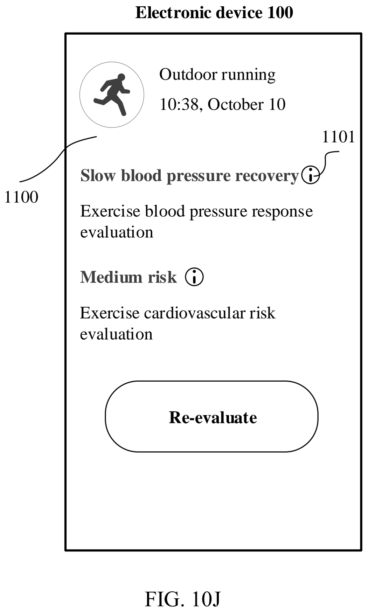

In some embodiments, the electronic device 100 may receive an input operation (for example, a tap) performed by the user on the option 1091. In response to the input operation, the electronic device 100 may display the display interface (for example, the user interface 1100 shown in FIG. 10J) of the exercise and health result. In some other embodiments, the electronic device 100 may send the collected physiological data and the exercise data to the electronic device 200 through the communication connection between the electronic device 100 and the electronic device 200. After receiving the physiological data and the exercise data from the electronic device 100, the electronic device 200 may obtain an exercise and health result of the user through analysis based on the physiological data and the exercise data by using an algorithm model. Then, the electronic device 200 may display a display interface of the exercise and health result.

For example, as shown in FIG. 10J, the electronic device 100 may display the user interface 1100, and the interface 1100 may include information related to blood pressure response evaluation and cardiovascular risk prediction. The blood pressure response evaluation indicates a blood pressure level evaluation result of the user in an entire exercise process. For example, "slow blood pressure recovery" may indicate that the blood pressure level of the user recovers slowly. The cardiovascular risk prediction may indicate an exercise-related cardiovascular risk evaluation result of the user. For example, "medium risk" may indicate that the cardiovascular risk evaluation result of the user is in a non-good state. The interface 1100 may further include a control 1101. The control 1101 may be used to trigger the electronic device 200 to display a display interface of a detailed exercise and health result.

As shown in FIG. 11A and FIG. 11B, the electronic device 100 may receive an input operation (for example, a tap) performed by the user on the control 1101. In response to the input operation, the electronic device 200 may display the display interface (for example, a user interface 1110 shown in FIG. 11B) of the detailed exercise and health result.

For example, as shown in FIG. 11B, the electronic device 200 may display the user interface 1110 of the detailed exercise and health result. The user interface 1110 may include blood pressure response evaluation, cardiovascular risk prediction, result interpretation information, a blood pressure control level, and exercise-related information. The blood pressure response evaluation indicates a blood pressure level evaluation result of the user in an entire exercise process. For example, "slow blood pressure recovery" may indicate that the blood pressure level of the user recovers slowly. The cardiovascular risk prediction may indicate an exercise-related cardiovascular risk evaluation result of the user. The cardiovascular risk evaluation result may indicate, as a whole, related information of an exercise and health risk obtained by the user by completing exercise-related cardiovascular risk evaluation at a moment. In some embodiments, the indication information may present the exercise and health risk of the user in a form of score and/or risk level (for example, a low risk, a medium risk, or a high risk). For example, the "medium risk" may indicate that the cardiovascular risk evaluation result of the user is in a non-good state. The result interpretation information may indicate interpretation information of the blood pressure response evaluation and the cardiovascular risk prediction, and an exercise and health suggestion. The blood pressure control level indicates a blood pressure level change status of the user in the entire exercise process. The user interface 1110 may provide the blood pressure level change status of the user in the entire exercise process and numerical display of the blood pressure level change status. For example, SBP and DBP ranges, a maximum increase/decrease value, and a recovery rate are displayed. The exercise-related information may include items such as a distance, exercise duration, an average pace, an average heart rate, and a maximum heart rate. It may be understood that display of the item is merely an example, and is not limited thereto. Content of the item may be related to the exercise mode of the user.

In some embodiments, different exercise-related cardiovascular risk evaluation results indicated by cardiovascular risk prediction correspond to different colors for differentiated display. For example, "low risk" corresponds to green, "medium risk" corresponds to yellow, and "high risk" corresponds to red. It is easy to understand that when the cardiovascular risk evaluation is "high risk", a display interface of a corresponding exercise and health result may further include related prompt information.

In this embodiment of this application, experiments are performed on some samples, and an experiment process and result are as follows:

### Sample 1:

Basic information input: The user is a healthy person and has exercise habits.

Before first exercise: No abnormality is detected for each indicator.

During first exercise: No abnormality is detected for each indicator.

After first exercise: When the user measures blood pressure, heartbeat intervals are found to be irregular for two consecutive times of blood pressure measurement, the electronic device 100 automatically reminds the user to measure an ECG, and submits an ECG report to a remote doctor for a manual interpretation. A ventricular trigeminy is detected. In some embodiments, the electronic device 100 may analyze the ECG report by using an artificial intelligence (artificial intelligence, AI) model. For example, the electronic device 100 may send the ECG report to a cloud for AI analysis, or send the ECG report to another electronic device (for example, the electronic device 200) connected in a near field for AI analysis, or perform AI analysis by the electronic device 100.

Risk evaluation: Among risk factors, a risk level of heart rate variability is high, and a comprehensive risk level is medium.

Suggestion: Total exercise duration is 22 minutes, heart rate reserve is at 18% in a zone 1, and heart rate reserve is at 82% in a zone 2. It is recommended that the user needs to reduce exercise intensity moderately.

In this embodiment of this application, after receiving the physiological data and the exercise data from the electronic device 100, the electronic device 200 may obtain an exercise and health result of the user through analysis based on the physiological data and the exercise data by using an algorithm model. Then, the electronic device 200 may display a display interface of an exercise and health report.

With reference to FIG. 12A to FIG. 12C, the following describes the method provided in this embodiment of this application by using an exercise and health report of the first exercise in an experimental sample 1 as an example.

**FIG. 12A to FIG. 12C** **show an example of display interfaces of the exercise and health report of the first exercise in the experimental sample 1.**

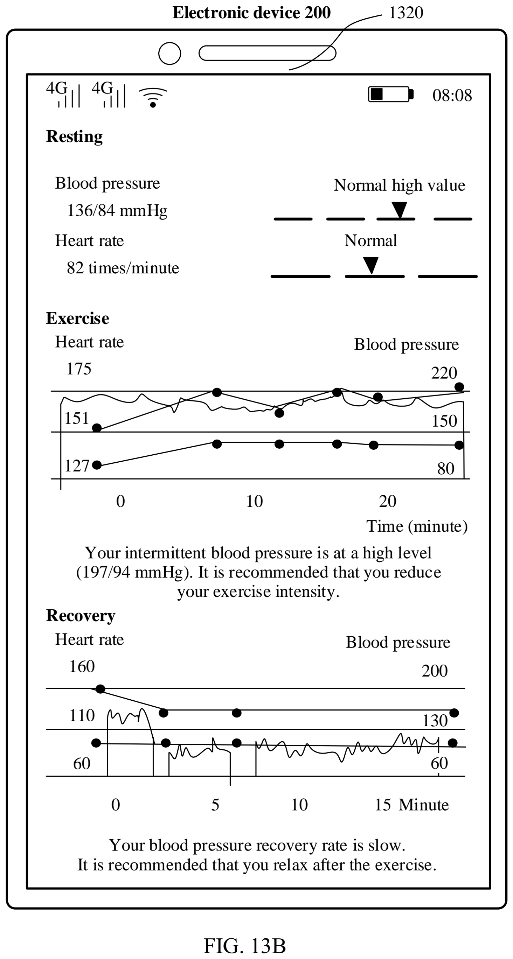

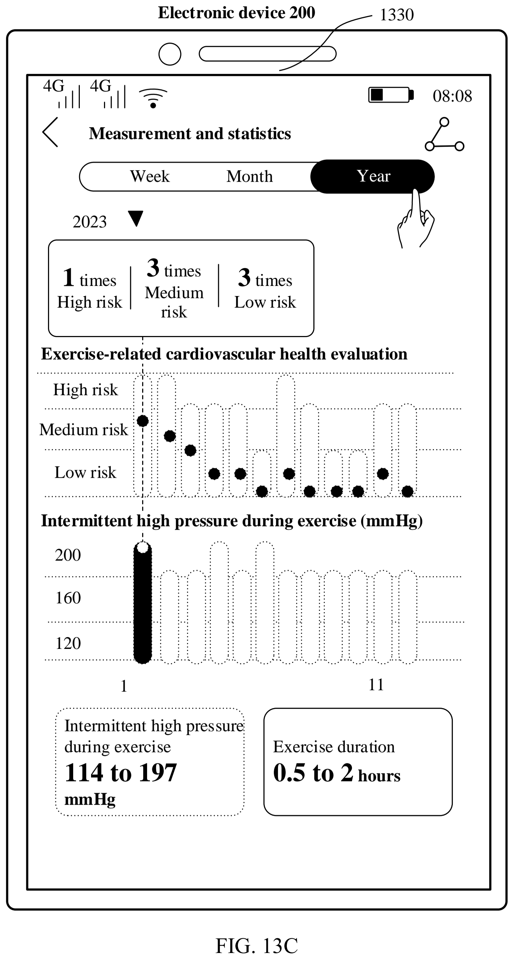

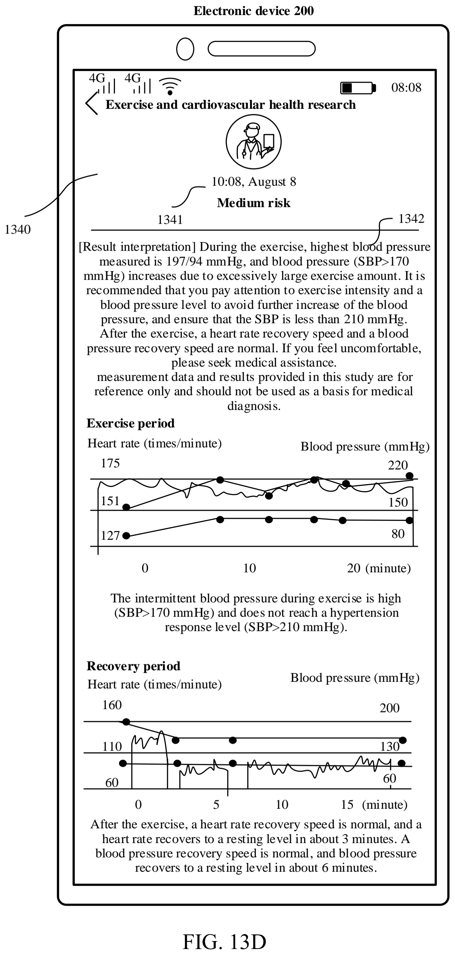

The exercise and health report may be generated based on the exercise and health result of the user. The display interface of the exercise and health report may include but is not limited to the user interface 1210 shown in FIG. 12A, the user interface 1220 shown in FIG. 12B, the user interface 1230 shown in FIG. 12C, and a user interface 1340 shown in FIG. 13D.

For example, as shown in FIG. 12A, the electronic device 200 may display the user interface 1210. The user interface 1210 may include related information, for example, indication information 1211, that indicates, as a whole, the exercise and health risk obtained by the user by completing the exercise-related cardiovascular risk evaluation. In some embodiments, the indication information may display the exercise and health risk of the user in a form of score and/or risk level (for example, a low risk, a medium risk, or a high risk). "Medium risk" in the indication information 1211 may indicate that the exercise and health result of the user is in a non-good state. The user interface 1210 may include related information that indicates a risk factor that affects the exercise and health result of the user, for example, indication information 1212. The risk factor indicated by the indication information 1212 may include but is not limited to intermittent blood pressure during exercise, a blood pressure recovery speed, a heart rate recovery speed, and heart rate variability. The intermittent blood pressure during exercise, the blood pressure recovery speed, the heart rate recovery speed, and the heart rate variability may respectively indicate a blood pressure status of the user when the user pauses the exercise during the exercise, a post-exercise blood pressure recovery status, a post-exercise heart rate recovery status, and a variation in a time interval between heartbeats. It is easy to understand that in this embodiment of this application, only the risk factor on the user interface 1210 shown in FIG. 12A is used as an example for description. In some embodiments, a quantity of risk factors may alternatively be less or more than a quantity of risk factors on the user interface 1210 shown in FIG. 12A, and the quantity of risk factors is related to types of the collected pre-exercise physiological data, the collected in-exercise physiological data, and the collected post-exercise physiological data. The user interface 1210 may include related information that indicates a result interpretation of the exercise and health result, for example, indication information 1213. The result interpretation information indicated by the indication information 1213 may be an interpretation of the risk factor indicated by the indication information 1212, so that the user understands the risk factor indicated by the indication information 1212. The user interface 1210 may include an option 1214. The option 1214 may be used to trigger entering the user interface 1220.

In some embodiments, the electronic device 200 may receive an input operation (for example, a tap) performed by the user on the option 1214. In response to the input operation, the electronic device 200 may display the user interface 1220 shown in FIG. 12B.

For example, as shown in FIG. 12B, the electronic device 200 may display the user interface 1220. The user interface 1220 may provide a physical health status like blood pressure and a heart rate when the user is resting (before the exercise), a physical health status like blood pressure and a heart rate during the exercise, and a physical health status like blood pressure and a heart rate during the exercise recovery (after the exercise). The physical health status like the blood pressure and the heart rate of the user during resting may be displayed in a numerical manner, and a level of the physical health status may be evaluated. For example: The user interface 1220 shown in FIG. 12B shows that blood pressure of the user in the experimental sample 1 during resting is 124/74 mmHg, which is at a normal level, and a heart rate is 72 bpm, which is also at a normal level. The physical health status like the blood pressure and the heart rate of the user during the exercise and during the exercise recovery may be displayed in a graph manner, and the electronic device 200 may display a result interpretation and a suggestion for the physical health status during the exercise and after the exercise. This suggestion is related to basic user information and an interpretation of the user's health status at different stages. For example, a suggestion displayed in a first case is "It is recommended to keep aerobic exercise", basic user information corresponding to the suggestion includes that the user is over 35 years old and plays badminton every week, and a physical health status at different stages corresponding to the suggestion is interpreted as that after 5 minutes of running, a heart rate is 110 times/minute, blood pressure exceeds 190 mmHg, and a hospital examination result is slight arterial stenosis. A suggestion displayed in a second case is "It is recommended to seek medical assistance for medical examination", basic user information corresponding to the suggestion includes that the user is over 28 years old, and a physical health status at different stages corresponding to the suggestion is interpreted as that resting blood pressure is 110 mmHg, and blood pressure abnormally decreases to less than 60 mmHg after 5 minutes of jogging. A suggestion displayed in a third case is "It is recommended to seek medical assistance for medical examination", basic user information corresponding to the suggestion includes that the user is over 35 years old and runs every week, and a physical health status at different stages corresponding to the suggestion is interpreted as a ventricular trigeminy occurs for long time after half an hour of running.

Refer to FIG. 10G to FIG. 10H. When the electronic device 100 detects a plurality of suspected irregular heartbeats of the user, the user may choose to measure an ECG. An ECG result and a related interpretation may be displayed on the user interface 1220, to help the user learn of a potential risk in time. This helps the user take an active intervention measure, to avoid occurrence of a cardiovascular risk event caused by excessive exercise intensity, enhancing user experience.

In some embodiments, as shown in FIG. 12C, the electronic device 200 may display the user interface 1230 as a display interface of the exercise and health report of the experimental sample 1. The user interface 1230 may include related information that indicates to collect statistics on an exercise and health result obtained by the user by completing exercise-related cardiovascular risk evaluation within a long period of time (for example, a week/month/year), for example, indication information 1231. The indication information 1231 may include a time unit control 1231a and a time selection control 1231b. The time unit control 1231a may be used by the user to select a time unit for collecting statistics on the exercise and health result. The time selection control 1231b may be used by the user to select a time range for collecting statistics on the exercise and health result.

Before second exercise: The electronic device 100 reminds the user of an evaluation suggestion of a previous exercise, and no abnormality is detected for each indicator.

During second exercise: No abnormality is detected for each indicator, and a heart rate is in a zone 1 (92%) of heart rate reserve and a zone 2 (8%) of heart rate reserve.

After second exercise: No abnormality is detected for each indicator.

Risk evaluation: No high-level risk factor is detected, and a comprehensive risk level is low.

Suggestion: Keep it up and may increase exercise intensity with caution.

According to the method, the user can detect arrhythmia induced by exercise in time, avoid a more serious myocardial ischemia attack symptom, and provide a reminder and a suggestion in time.

### Sample 2:

Basic information input: A user finds an abnormality during a medical examination in a hospital. An electrocardiogram indicates that an abnormality occurs in an ST segment. A computed tomography (computed tomography, CT) of a heart indicates that there is a slight coronary arterial stenosis. A carotid ultrasound indicates that there is carotid intima-media roughness. A doctor's advice is to exercise more, pay attention to the diet, and moderately lose weight.

Before first exercise: Resting blood pressure is normal and a heart rate is normal.

During first exercise: The user stops running for 5 minutes in a zone 1 of heart rate reserve to measure blood pressure. A measurement result is that systolic blood pressure (systolic blood pressure, SBP) is greater than 190 mmHg.

After first exercise: Take a rest immediately and wait for 10 minutes until blood pressure returns to normal.

Risk evaluation: Among risk factors, a risk level of in-exercise hypertension response is high, and a comprehensive risk level is high.

Suggestion: It is recommended to reduce exercise intensity moderately.

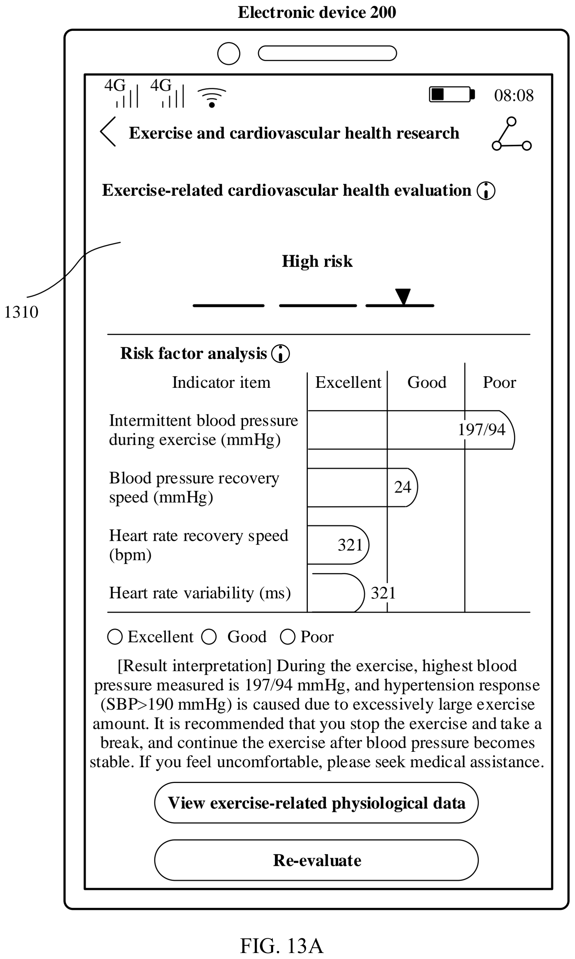

FIG. 13A to FIG. 13C are diagrams of user interfaces of a method according to an embodiment of this application by using an exercise and health report of a first exercise in an experimental sample 2 as an example. For descriptions of a user interface 1310, a user interface 1320, and a user interface 1330 shown in FIG. 13A to FIG. 13C, refer to related content in the embodiment in FIG. 12A to FIG. 12C. Details are not described herein again.

In some embodiments, as shown in FIG. 13D, the electronic device 200 may display the user interface 1340 as a display interface of the exercise and health report of the experimental sample 2.

**FIG. 13D** **shows an example of display interfaces of the exercise and health report of the first exercise in the experimental sample 2.**

For example, as shown in FIG. 13D, the electronic device 200 may display the user interface 1340. The user interface 1340 may include related information, for example, indication information 1341, that indicates, as a whole, the exercise and health risk obtained by the user by completing the exercise-related cardiovascular risk evaluation at a moment. In some embodiments, the indication information may display the exercise and health risk of the user in a form of score and/or risk level (for example, a low risk, a medium risk, or a high risk). "Medium risk" in the indication information 1341 may indicate that the exercise and health result of the user is in a non-good state. The user interface 1340 may provide a physical health status like blood pressure and a heart rate during the exercise, and a physical health status like blood pressure and a heart rate during the exercise recovery (after the exercise). The physical health status like the blood pressure and the heart rate of the user during the exercise and during the exercise recovery may be displayed in a graph manner, and the electronic device 200 may display a result interpretation and a suggestion for the physical health status during the exercise and after the exercise. The user interface 1340 may further include related information that indicates a result interpretation of the exercise and health result, for example, indication information 1342. The result interpretation information indicated by the indication information 1342 may be an interpretation of the physical health status like the blood pressure and the heart rate of the user during the exercise and during the exercise recovery.

Before second exercise: Resting blood pressure is normal and a heart rate is normal.

During second exercise: An exercise mode is changed from running to badminton (doubles), and a heart rate is lower than a zone 1 of heart rate reserve or is in the zone 1 of heart rate reserve for short time. No abnormality is detected during the exercise interval.

After second exercise: No abnormality is detected for each indicator.

Risk evaluation: No high-level risk factor is detected, and a comprehensive risk level is low.

Suggestion: Keep it up and may increase exercise intensity with caution if there is no hypertension during the exercise.

### Sample 3:

Basic information input: female, thin, mild anemia, no exercise habits, feeling uncomfortable during the exercise, and unaware of cardiovascular risks related to exercise.

Before first exercise: Blood pressure and a heart rate are normal.

During first exercise: SBP decreased by more than 30 mmHg compared with that before the exercise when blood pressure is measured after running for 5 minutes (jogging) in the zone 1 of heart rate reserve. The user feels dizziness, chest tightness, and nausea, and cannot stand stable.

After first exercise: Stop exercise immediately and rest in a chair. After 10 minutes, the blood pressure return to normal and walking gait return to normal.

Risk evaluation: A risk factor is high, a risk level of in-exercise blood pressure is high, and a comprehensive risk level is high.

Suggestion: It is recommended to change from jogging to fast walking and evaluate a blood pressure level during the exercise.

Before second exercise: A previous evaluation result is displayed again. No abnormality is detected for each indicator this time.

During second exercise: An exercise mode is changed from jogging to fast walking, and no abnormality is detected during blood pressure measurement at an exercise interval.

After second exercise: No abnormality is detected for each indicator.

Risk evaluation: No high-level risk factor is detected, and a comprehensive risk level is low.

Suggestion: Keep it up and may increase exercise intensity with caution if there is no hypotension risk during the exercise.

### Sample 4:

Basic information input: an elderly over 65 years old, many basic diseases, no exercise habits except for housework and shopping, rarely go out, easy to fatigue, and poor physical strength. A doctor's advice is to take medicines on time and take moderate outdoor activities, such as walking.

Before first exercise: SBP is 154 mmHg, diastolic blood pressure (diastolic blood pressure, DBP) is 92 mmHg, and a heart rate is normal.

During first exercise: Gait stability identified based on a wrist posture is poor and lower than a threshold after 5 minutes (5.8 km/h) of walking. The device reminds that there is a risk of falling. The user stops to measure blood pressure. SBP is 182 mmHg, and DBP is 99 mmHg. The blood pressure is high and is close to a threshold. The user rests for a while and changes to walk slowly (4.2 km/h). After 20 minutes, the user stops exercising.

After first exercise: No abnormality is detected for each indicator.

Risk evaluation: Among risk factors, an in-exercise falling risk is medium, a risk level of hypertension response is medium, and a comprehensive risk level is medium.

Suggestion: It is recommended to walk slowly at a speed not higher than 5.8 km/h and pay attention to an in-exercise blood pressure level.

Before second exercise: No abnormality is detected for each indicator.

During second exercise: Walk slowly. Exercise duration is gradually extended from 20 minutes to 40 minutes.

After second exercise: No abnormality is detected for each indicator.

Risk evaluation: No high-level risk factor is detected, and a comprehensive risk level is low.

Suggestion: Keep it up and may increase exercise intensity with caution if there is no risk of falling or hypertension.

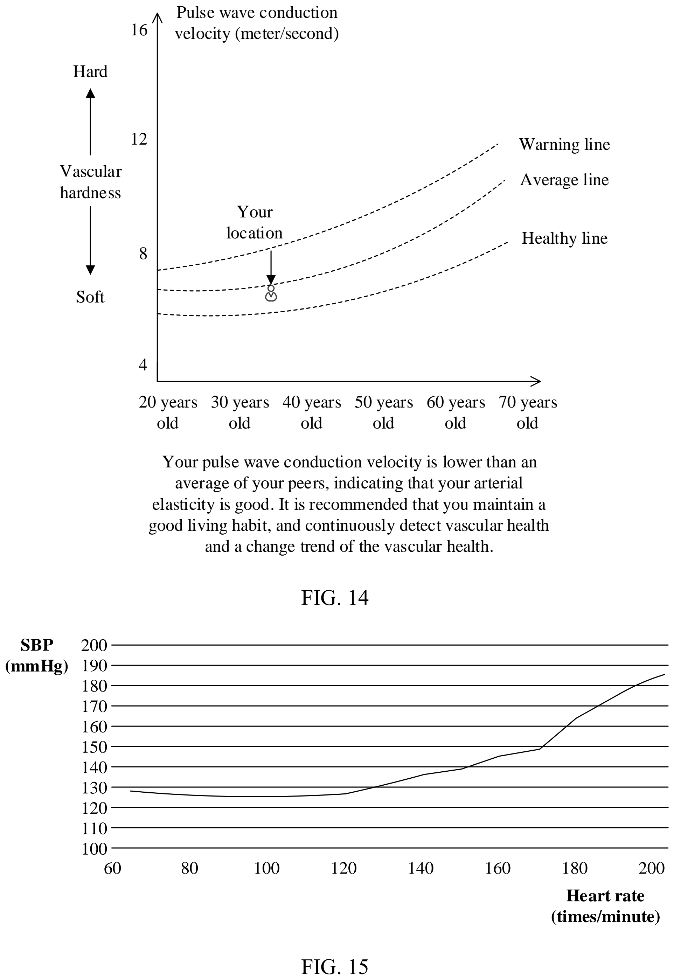

In some embodiments, the electronic device 200 may draw, based on big data of a crowd, a relationship curve between an indicator of the user and an age under same exercise intensity, and indicate a location of the indicator of the user within population distribution. The exercise intensity may be represented by heart rate reserve/pace/metabolic equivalent of task/tread frequency/power/accumulated exercise amount/heart rate reserve when the exercise stops/blood pressure when the exercise stops, or the like. An indicator of the user may be, for example, an SBP/DBP/pulse pressure difference (pulse pressure, PP)/heart rate level, or may be, for example, a heart rate/SBP/DBP recovery speed, or may be, for example, a pulse wave conduction velocity. The pulse wave conduction velocity is used as an example, as shown in FIG. 14. Under specific exercise intensity, as the user ages, a vascular hardness of the user usually increases. In other words, the pulse wave conduction velocity becomes faster. This means that a cardiovascular health status of the user becomes worse. Under same exercise intensity (for example, a same pace), the pulse wave conduction velocity of the user shown in FIG. 14 is lower than an average value but higher than a healthy value compared with a peer. The electronic device 200 may interpret a statistical chart shown in FIG. 14 and provide a suggestion (for example, "Your arterial elasticity is good. It is recommended that you maintain a good living habit, and continuously detect vascular health and a change trend of the vascular health"). Optionally, the electronic device 200 may send data to the electronic device 100, and the electronic device 100 displays the statistical chart. In this way, the statistical chart is displayed, so that the user can intuitively learn of a health level of a cardiovascular system of the user.

In some embodiments, the electronic device 200 may draw a physiological data change trend chart of the user at different exercise intensity (for example, may be represented by a heart rate) based on a plurality of pieces of exercise data of the user. Blood pressure is used as an example, as shown in FIG. 15. Generally, as the exercise intensity increases, the blood pressure of the user gradually increases. The electronic device 200 may give a suggestion to the user based on the trend chart shown in FIG. 15 (for example, "A probability of abnormal blood pressure in an XX pace range and an XX heart rate range is low. It is recommended to control the pace within the XX range and control the heart rate within the XX range during the exercise"). Optionally, the electronic device 200 may send data to the electronic device 100, and the electronic device 100 displays the trend chart. In this way, the trend chart is displayed, so that the user can further learn of an exercise capability of the cardiopulmonary system of the user. This helps the user moderately control exercise intensity of the user, to ensure exercise effectiveness.

In some possible implementations, to obtain a multi-dimensional cardiovascular health evaluation result of the user, the electronic device 100 may measure blood pressure a plurality of times after detecting that the exercise of the user ends. For example, the electronic device 100 measures blood pressure three times after the exercise of the user ends. The electronic device 100 may measure blood pressure three times after the exercise of the user ends, and obtain first post-exercise blood pressure, second post-exercise blood pressure, and third post-exercise blood pressure. Specifically, the electronic device 100 may measure blood pressure once immediately after the exercise of the user ends, to obtain the first post-exercise blood pressure. Then, the electronic device 100 may measure blood pressure of the user after a first time period after the exercise of the user ends, to obtain the second post-exercise blood pressure. Then, the electronic device 100 may measure blood pressure of the user after a second time period after the exercise of the user ends, to obtain the third post-exercise blood pressure. For example, the first time period may be 3 minutes after the exercise of the user ends, and the second time period may be 6 minutes after the exercise of the user ends. The first time period and the second time period are not specifically limited in this embodiment of this application.

In some embodiments, the first post-exercise blood pressure, the second post-exercise blood pressure, and the third post-exercise blood pressure may be collectively referred to as post-exercise blood pressure.

In a possible implementation, the post-exercise blood pressure is determined based on a pulse oscillation wave signal that is of the user and that is measured by the electronic device 100 after the exercise of the user ends and a resting blood pressure result that is of the user and that is measured by the electronic device 100 before the exercise of the user. The resting blood pressure result may include systolic blood pressure, diastolic blood pressure, and/or a heart rate of the user. The resting blood pressure result may be obtained by the electronic device 100 through measurement before the current exercise of the user. Optionally, the resting blood pressure result may alternatively be resting blood pressure data that is historically measured by the user and that is stored by the electronic device 100.

In a possible implementation, the blood pressure response evaluation (which may also be referred to as exercise blood pressure response evaluation) includes hypertension response evaluation, hypotension response evaluation, and blood pressure recovery evaluation. The hypertension response evaluation is obtained based on the pre-exercise blood pressure, the in-exercise blood pressure (or referred to as intermittent blood pressure during exercise), or the post-exercise blood pressure (one or more of the first post-exercise blood pressure, the second post-exercise blood pressure, and the third post-exercise blood pressure).

The hypotension response evaluation may be obtained based on the pre-exercise blood pressure and the first post-exercise blood pressure. Optionally, the hypotension response evaluation may be obtained based on the pre-exercise blood pressure, the first post-exercise blood pressure, and the in-exercise blood pressure. In other words, if the pre-exercise blood pressure or the first post-exercise blood pressure is missing, the electronic device 100 cannot obtain the hypotension response evaluation.

The blood pressure recovery evaluation may be used to evaluate whether blood pressure recovery of the user is slow after the exercise ends. The blood pressure recovery evaluation may be obtained based on the pre-exercise blood pressure and the third post-exercise blood pressure. Optionally, the blood pressure recovery evaluation may be obtained based on the pre-exercise blood pressure, the third post-exercise blood pressure, and the in-exercise blood pressure. In other words, if the pre-exercise blood pressure or the third post-exercise blood pressure is missing, the electronic device 100 cannot obtain the blood pressure recovery evaluation.

In a possible implementation, cardiovascular risk prediction (which may also be referred to as exercise cardiovascular risk evaluation) may be obtained based on pre-exercise physiological data and post-exercise physiological data. Optionally, the cardiovascular risk prediction may be obtained based on pre-exercise physiological data, post-exercise physiological data, and in-exercise physiological data. The pre-exercise physiological data may include the pre-exercise blood pressure, the post-exercise physiological data may include the first post-exercise blood pressure, the second post-exercise blood pressure, and the third post-exercise blood pressure. The in-exercise physiological data may include the in-exercise blood pressure.

In conclusion, the foregoing embodiments are merely intended for describing the technical solutions of this application, but not for limiting this application. Although this application is described in detail with reference to the foregoing embodiments, a person of ordinary skill in the art should understand that they may still make modifications to the technical solutions described in the foregoing embodiments or make equivalent replacements to some technical features thereof, without departing from the scope of the technical solutions of embodiments of this application.

It should be understood that sequence numbers of the foregoing processes do not mean execution sequences in various embodiments of this application. The execution sequences of the processes should be determined according to functions and internal logic of the processes, and should not be construed as any limitation on the implementation processes of embodiments of this application.

According to the context, the term "when" used in the foregoing embodiments may be interpreted as a meaning of "if", "after", "in response to determining", or "in response to detecting". Similarly, according to the context, the phrase "when it is determined that..." or "if (a stated condition or event) is detected" may be interpreted as a meaning of "if it is determined that...", "in response to determining...", "when (a stated condition or event) is detected", or "in response to detecting (a stated condition or event)".

All or some of the foregoing embodiments may be implemented through software, hardware, firmware, or any combination thereof. When software is used to implement the foregoing embodiments, all or some of the foregoing embodiments may be implemented in a form of a computer program product. The computer program product includes one or more computer instructions. When the computer program instructions are loaded and executed on the computer, the procedure or functions according to embodiments of this application are all or partially generated. The computer may be a general-purpose computer, a dedicated computer, a computer network, or other programmable apparatuses. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by a computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

A person of ordinary skill in the art may understand that all or some of the procedures of the methods in embodiments may be implemented by a computer program instructing related hardware. The program may be stored in a computer-readable storage medium. When the program is run, the procedures of the methods in embodiments are performed. The foregoing storage medium includes any medium that can store program code, such as a ROM, a random access memory RAM, a magnetic disk, or an optical disc.

## Claims

1. A detection method, wherein the method comprises:
collecting, by the first electronic device, pre-exercise physiological data before a user starts a first exercise;
collecting, by the first electronic device, in-exercise physiological data in a process in which the user performs the first exercise;
collecting, by the first electronic device, post-exercise physiological data after the first exercise of the user ends; and
obtaining, by the first electronic device, an exercise and health result of the user based on the pre-exercise physiological data, the in-exercise physiological data, and the post-exercise physiological data, and outputting the exercise and health result.

2. The method according to claim 1, further comprising:
displaying, by the first electronic device, a first report used to describe the exercise and health result, and sending the exercise and health result to a second electronic device, for the second electronic device to display a second report used to describe the exercise and health result, wherein content of the first report is less than content of the second report.

3. The method according to claim 2, wherein the first electronic device comprises a wearable device, and a display of the second electronic device is larger than a display of the first electronic device.

4. The method according to any one of claims 1 to 3, further comprising:
displaying, by the first electronic device, a first prompt message in the process in which the user performs the first exercise, wherein the first prompt message indicates a health abnormality in the process in which the user performs the first exercise, and the first prompt message is generated based on the in-exercise physiological data, or is generated based on the pre-exercise physiological data and the in-exercise physiological data.

5. The method according to claim 4, wherein the health abnormality comprises one or more of the following: an abnormally high blood pressure, an abnormally low blood pressure, a retarded reaction, arrhythmia, an abnormal ST segment change, and an abnormal blood oxygen decrease.

6. The method according to any one of claims 1 to 5, wherein the exercise and health result comprises a blood pressure evaluation result, and the blood pressure evaluation result is generated based on the pre-exercise physiological data, the in-exercise physiological data, and the post-exercise physiological data, or is generated based on the pre-exercise physiological data and the post-exercise physiological data.

7. The method according to any one of claims 1 to 6, wherein the exercise and health result comprises cardiovascular risk prediction, and the cardiovascular risk prediction is generated based on two or three of the pre-exercise physiological data, the in-exercise physiological data, and the post-exercise physiological data, and one or more of an exercise mode, a pre-exercise questionnaire, and a post-exercise questionnaire.

8. The method according to any one of claims 1 to 7, wherein the process in which the user performs the first exercise comprises an exercise interval of the user performing the first exercise.

9. The method according to any one of claims 1 to 8, wherein before collecting the pre-exercise physiological data, the method further comprises:
obtaining, by the first electronic device, an exercise mode of the user, and selecting, based on the exercise mode, an algorithm model used to analyze the exercise and health result, wherein
the exercise mode comprises the first exercise; and
obtaining, by the first electronic device, the exercise and health result of the user based on the pre-exercise physiological data, the in-exercise physiological data, and the post-exercise physiological data specifically comprises:
analyzing, by the first electronic device, the pre-exercise physiological data, the in-exercise physiological data, and the post-exercise physiological data by using the algorithm model, to obtain the exercise and health result of the user.

10. The method according to claim 9, wherein obtaining, by the first electronic device, the exercise mode of the user specifically comprises:
obtaining, by the first electronic device, the exercise mode based on an input of the user;
or
collecting, by the first electronic device, exercise data of the user when the user starts the first exercise, and then determining the exercise mode based on the exercise data.

11. The method according to any one of claims 1 to 10, further comprising:
displaying, by the first electronic device, a first user interface if it is detected that the in-exercise physiological data deviates from normal physiological data of the user, wherein the first user interface comprises a second prompt, the second prompt indicates a risk that exists when the user performs the first exercise, and the normal physiological data is a reference value that is of physiological data of the user during the first exercise and that is determined based on the pre-exercise physiological data.

12. The method according to any one of claims 1 to 11, wherein the exercise and health result further comprises first evaluation, and the first evaluation indicates an exercise suggestion for the user to perform the first exercise.

13. The method according to claim 12, further comprising:
obtaining, by the first electronic device based on the exercise data in the first exercise process and the in-exercise physiological data, exercise and health results obtained when the user is in different exercise states in the process of performing the first exercise, wherein the first evaluation specifically indicates an exercise status that is suitable for or not suitable for the user in the different exercise states.

14. The method according to any one of claims 1 to 13, wherein the method further comprises:
obtaining, by the first electronic device based on historical physiological data and historical exercise data, an exercise suggestion before the first exercise starts, wherein the historical physiological data comprises physiological data collected before a previous exercise of the first exercise starts, physiological data collected in a process of the previous exercise, and physiological data collected after the previous exercise ends, the historical exercise data comprises exercise data of the previous exercise, the exercise suggestion before the first exercise starts comprises second evaluation, and the second evaluation indicates whether the user is suitable for or not suitable for the first exercise.

15. The method according to any one of claims 1 to 14, wherein the in-exercise physiological data comprises one or more of the following: a heart rate, blood pressure, an electrocardiogram, and blood oxygen; and
collecting, by the first electronic device, the in-exercise physiological data in the process in which the user performs the first exercise specifically comprises:
displaying, by the first electronic device, a second user interface in the process in which the user performs the first exercise, wherein the second user interface comprises exercise data and a heart rate that are in the process in which the user performs the first exercise; and
the second user interface further comprises an exercise pause control; and
detecting, by the first electronic device, an operation of tapping the exercise pause control by the user, suspending collecting the exercise data, and displaying a third user interface, wherein
the third user interface comprises one or more of the following options: a blood pressure measurement option, an electrocardiogram collection option, and a blood oxygen measurement option.

16. The method according to any one of claims 1 to 15, wherein "after the first exercise of the user ends" is specifically "before first duration after the first exercise of the user ends".

17. The method according to any one of claims 1 to 16, wherein the post-exercise physiological data comprises one or more of the following: a heart rate, blood pressure, an electrocardiogram, and blood oxygen; and
collecting, by the first electronic device, the post-exercise physiological data after the first exercise of the user ends specifically comprises:
the second user interface further comprises an exercise end control;
detecting, by the first electronic device, an operation of tapping the exercise end control by the user, ending collecting the exercise data, and displaying a fourth user interface, wherein
the fourth user interface comprises a countdown control, duration of the countdown control is set to second duration, and the second duration is less than or equal to the first duration; and
displaying, by the first electronic device, a fifth user interface when timing of the countdown control ends, wherein the fifth user interface comprises one or more of the following options: a blood pressure measurement option, a heart rate measurement option, an electrocardiogram collection option, and a blood oxygen measurement option.

18. The method according to any one of claims 1 to 17, wherein outputting the exercise and health result specifically comprises:
displaying one or more of the following risk factor items: a risk factor item evaluated based on the in-exercise physiological data and a risk factor item evaluated based on the post-exercise physiological data; and
displaying a comprehensive risk level of performing the first exercise by the user, wherein the comprehensive risk level is determined based on a risk level of the risk factor item evaluated based on the in-exercise physiological data and a risk level of the risk factor item evaluated based on the post-exercise physiological data.

19. An electronic device, wherein the electronic device comprises a processor and a memory, the memory is coupled to the processor, the memory is configured to store computer program code, the computer program code comprises computer instructions, and when the processor executes the computer instructions, the electronic device is enabled to perform the method according to any one of claims 1 to 18.

20. A computer storage medium, wherein the computer storage medium stores a computer program, the computer program comprises program instructions, and when the program instructions are run on an electronic device, the electronic device is enabled to perform the method according to any one of claims 1 to 18.
